# EUROPEAN PATENT SPECIFICATION

(11) **EP 2 768 986 B1**
(45) Date of publication and mention of the grant of the patent: **27.06.2018**
(21) Application number: 12852462.6
(22) Date of filing: 20.10.2012
(51) Int. Cl.: C12Q 1/68

(54) **PLASMA MICRORNAS FOR THE DETECTION OF EARLY COLORECTAL CANCER**
PLASMA-MIRNA FÜR DEN NACHWEIS VON KOLOREKTALKARZINOM IM FRÜHSTADIUM
MICROARN DU PLASMA POUR LA DÉTECTION DU CANCER COLORECTAL PRÉCOCE

(30) Priority: 21.10.2011 US 201161550148 P
(43) Date of publication of application: 27.08.2014
(60) Divisional application: 15158185.7
(73) Proprietor: Hospital Clínic de Barcelona, 08036 Barcelona (ES); Centro de Investigación Biomédica en Red de Enfermedades Hepáticas y Digestivas (CIBEREHD), 08036 Barcelona (ES)
(72) Inventor: GIRONELLA I COS, Meritxell, E-08190 Sant Cugat del Valles (ES); LOZANO SALVATELLA, Juan, Jose, E-08023 Barcelona (ES); CASTELLS I GARANGOU, Antoni, E-08109 Sant Cugat del Valles (Barcelona) (ES); GIRALDEZ, Maria Dolores, E-08022 Barcelona (ES)
(74) Representative: Hoffmann Eitle
(86) International application number: PCT/IB2012/003035
(87) International publication number: WO 2013/093635

(56) References cited:
- WO-A1-2011/076142
- WO-A1-2011/160585
- WO-A2-2009/111643
- SYLVAIN PRADERVAND ET AL: "Concordance among digital gene expression, microarrays, and qPCR when measuring differential expression of microRNAs", BIOTECHNIQUES, vol. 48, no. 3, 1 March 2010 (2010-03-01), pages 219-222, XP055012457, ISSN: 0736-6205, DOI: 10.2144/000113367
- Y. KOGA ET AL: "MicroRNA Expression Profiling of Exfoliated Colonocytes Isolated from Feces for Colorectal Cancer Screening", CANCER PREVENTION RESEARCH, vol. 3, no. 11, 19 October 2010 (2010-10-19), pages 1435-1442, XP55067024, ISSN: 1940-6207, DOI: 10.1158/1940-6207.CAPR-10-0036
- GE YU ET AL: "Prognostic values of the miR-17-92 cluster and its paralogs in colon cancer", JOURNAL OF SURGICAL ONCOLOGY, vol. 106, no. 3, 7 November 2011 (2011-11-07), pages 232-237, XP55067086, ISSN: 0022-4790, DOI: 10.1002/jso.22138

## Description

### CROSS-REFERENCE TO RELATED APPLICATIONS

This application claims priority to U.S. Provisional Application Serial No. 61/550,148, filed October 21, 2011, the entire contents of which are incorporated herein by reference.

### TECHNICAL FIELD OF THE INVENTION

The present invention relates in general to the field of colorectal cancer detection, and more particularly, to plasma microRNAs for the detection of early colorectal cancer.

### STATEMENT OF FEDERALLY FUNDED RESEARCH

None.

### INCORPORATION-BY-REFERENCE OF MATERIALS FILED ON COMPACT DISC

None.

### BACKGROUND OF THE INVENTION

Without limiting the scope of the invention, its background is described in connection with colorectal cancers.

U.S. Patent Application No. 20100317533 (Lou et al. 2010) provides a panel of biomarkers of tumor metastasis comprising any two of carbonic anhydrase-9 (CAIX), vascular endothelial growth factor C (VEGF-C), ephrin A5 (EFNA5), eph receptor B2 (EPHB2), transforming growth factor beta 3 (TGF-β3), pyruvate dehydrogenase kinase isoenzyme-3 (PDK3), carbonic anhydrase-12 (CAXII), keratin 14 (KRT14), hypoxia inducible factor 1 alpha subunit (HIF-1α), or tenascin C (TNC). CAIX, VEGF-C, EFNA5, EPHB2, TGF-β3 or PDK3 may be indicators of moderate metastatic potential, while CAXII, KRT14, HIF-1α, or TNC may be indicators of high metastatic potential. There is also provided a method of determining risk of tumor metastasis using the aforementioned biomarkers is also provided. The biomarkers may be used in diagnosis, prognosis, treatment selection, or to test putative therapeutics. The biomarkers may be used to assess malignancies or cancers having hypoxic regions, such as breast cancer.

U.S. Patent Application No. 20100120898 (Croce et al. 2010) discloses methods and compositions for the diagnosis, prognosis and treatment of Hepatocellular carcinoma (HCC). Also provided are methods of identifying anti-HCC agents. The Croce application provides a method diagnosing whether a subject has, or is at risk for developing, hepatocellular carcinoma (HCC), comprising measuring the level of at least one miR gene product in a test sample from the subject, wherein an alteration in the level of the miR gene product in the test sample, relative to the level of a corresponding miR gene product in a control sample, is indicative of the subject either having, or being at risk for developing, HCC.

United States Patent No. 7,939,255, issued to Chung is directed to diagnostic methods for colorectal cancer. Briefly, the patent discloses a diagnostic method and a kit for prognosis assessment of colorectal cancer (CRC) with a tumor suppressor gene to be used for diagnosis of colorectal cancer (CRC), wherein the method comprises: identifying recurrently altered regions (RAR) on a chromosome; and detecting genomic alterations in the RAR. It is said that the invention makes it possible to perform early diagnosis as well as prognosis assessment for various cancers and tumors including colorectal cancer (CRC).

Publication No. WO2011076147, entitled, Plasma-Based Micro-RNA Biomarkers And Methods For Early Detection of Colorectal, filed by Li, a diagnostic kit of molecular markers in blood for diagnosing colorectal cancer, and/or monitoring the therapeutic effect for treating colorectal cancer is disclosed. The kit is said to comprise a plurality of nucleic acid molecules, and each nucleic acid molecule encodes a microRNA biomarker, wherein one or more of the plurality of nucleic acid molecules are differentially expressed in plasma of patient and healthy control, and the one or more differentially expressed nucleic acid molecules together represent a nucleic acid expression biomarker that is indicative for the presence of colorectal cancer. The invention is said to further provide corresponding methods using such nucleic acid expression biomarkers for identifying colorectal cancer as well as for preventing or treating such a condition. Finally, the invention provides a pharmaceutical composition for the prevention and/or treatment of colorectal cancer.

Publication No. WO2011076142, entitled, Compositions And Methods For MicroRNA Expression Profiling in Plasma of Colorectal, also filed by Li, is said to teach compositions and methods for microRNA (miRNA) expression profiling in plasma of colorectal cancer. In particular, the invention is said to relate to a diagnostic kit of molecular markers in blood for diagnosing colorectal cancer, monitoring the cancer therapy and/or treating colorectal cancer that includes a plurality of nucleic acid molecules, each nucleic acid molecule encoding a microRNA sequence, wherein one or more of the plurality of nucleic acid molecules are differentially expressed in plasma of colorectal cancer and healthy control plasma, and wherein the one or more differentially expressed nucleic acid molecules together represent a nucleic acid expression signature that is indicative for the presence of colorectal cancer. The invention is said to further relate to corresponding methods of using such nucleic acid expression signatures for identifying colorectal cancer as well as for preventing or treating such a condition. Finally, the invention is directed to a pharmaceutical composition for the prevention and/or treatment of colorectal cancer.

Publication No. WO2011088226, entitled, Detection Of Gastrointestinal Disorders, filed by, Christine, is said to teach methods and systems for characterizing a phenotype by detecting microRNAs, vesicles, or biomarkers that are indicative of disease or disease progress. The disease can be a gastrointestinal disorder, such as colorectal cancer. The microRNAs, vesicles, or biomarkers can be detected in a bodily fluid.

Publication No. WO2010004562, entitled, Methods And Compositions For Detecting Colorectal Cancer, filed by Baruch, is said to teach a method for conducting minimally-invasive early detection of colorectal cancer and/or of colorectal cancer precursor cells, by using microRNA molecules associated with colorectal cancer, as well as various nucleic acid molecules relating thereto or derived thereof.

Finally, Publication No. WO2011012136, entitled, A Method For Classifying A Human Cell Sample As Cancerous, filed by Fog, et al., is said to teach a method for discriminating between cancer and non-cancer samples is described. The method is said to comprise detecting the level of at least one microRNA (miR) selected from Mir- Group I consisting of: miR-21, miR-34a and miR-141, and detecting the level of at least one miR selected from Mir-Group Il consisting of: miR-126, miR-143 and miR-145 in a test cell sample and, comparing the level of expression of said selected miRs in the test cell sample with the level of expression of the same selected miRs in a previously recorded test set.

### SUMMARY OF THE INVENTION

In one embodiment the present invention includes a method for diagnosing or detecting colorectal neoplasia in a human subject comprising the steps of: obtaining one or more biological samples from the subject suspected of suffering from colorectal neoplasia; measuring an overall expression pattern or level of one or more microRNAs obtained from the one or more biological samples of the subject; and comparing the overall expression pattern of the one or more microRNAs from the biological sample of the subject suspected of suffering from colorectal neoplasia with the overall expression pattern of the one or more microRNAs from a biological sample of a normal subject, wherein the normal subject is a healthy subject not suffering from colorectal neoplasia, wherein overexpression of miR15b, or miR15b in combination with miR19a and miR19b, or miR19a and miR19b is indicative of colorectal cancer. In one aspect, the method further comprises the analysis of at least one of miR18a, miR29a, or miR335 as compared to expression from the normal subject is indicative of colorectal neoplasia. In another aspect, the method further comprises the analysis of at least one of miR29a, miR92a, or miR141. In another aspect, the one or more biological samples are selected from the group consisting of one or more biological fluids, a plasma sample, a serum sample, a blood sample, a tissue sample, or a fecal sample. In another aspect, the method is capable of detecting early CRC (I-II) as accurately as advanced CRC (stage II-III), right-sided tumors and left-sided lesions. In another aspect, the method comprises confidence interval that is 90, 91, 92, 93, 94, or 95% of greater. In another aspect, the method further comprises determining of the level of expression of microRNAs that are underexpressed in colorectal neoplasia are selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

In another aspect, the method further comprises determining of the level of expression of microRNAs that are overexpressed in colorectal neoplasia are selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p:11.0; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

In another aspect, the expression level of the one or more microRNAs is measured by microarray expression profiling, PCR, reverse transcriptase PCR, reverse transcriptase real-time PCR, quantitative real-time PCR, end-point PCR, multiplex end-point PCR, cold PCR, ice-cold PCR, mass spectrometry, in situ hybridization (ISH), multiplex in situ hybridization, or nucleic acid sequencing. In another aspect, the method is used for treating a patient at risk or suffering from colorectal neoplasia, selecting an anti-neoplastic agent therapy for a patient at risk or suffering from colorectal neoplasia, stratifying a patient to a subgroup of colorectal neoplasia or for a colorectal neoplasia therapy clinical trial, determining resistance or responsiveness to a colorectal neoplasia therapeutic regimen, developing a kit for diagnosis of colorectal neoplasia or any combinations thereof. In another aspect, the overall expression pattern or level of 4, 5, 6, 7, 8, 9, 10, 12, 15, 20, 25, 30, 35, 40, 45, 50, 55 or 60 microRNAs selected from Tables 2, 3, 4, and 5, wherein the microRNAs increase the specificity of the determination, diagnosis or detection of colorectal neoplasia. In another aspect, the method further comprises the step of using the overall expression pattern or level of microRNAs for prognosis, treatment guidance, or monitoring response to treatment of the colorectal neoplasia.

Yet another embodiment of the present invention includes a biomarker for colorectal neoplasia disease progression, metastasis or both wherein the biomarker comprises one or more microRNAs and a change in the overall expression of the one or more microRNAs in colorectal neoplasia cells obtained from a patient is indicative of colorectal neoplasia disease progression when compared to the overall expression of the one or more microRNAs expression in normal colorectal neoplasia cells or colorectal neoplasia cells obtained at an earlier timepoint from the same patient, wherein the overexpression of the combination of at miR19a and miR19b, or miR19a and miR19b and miR15b, is indicative of colorectal cancer. In one aspect, the method further comprises the analysis of one or more of the following microRNAs miR29a, miR92a, miR141, miR18a, miR19a, miR19b, miR15b, miR29a or miR335. In another aspect, the biomarker further comprises microRNAs that are underexpressed in colorectal neoplasia selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

In another aspect, the biomarker further comprises microRNAs that are overexpressed in colorectal neoplasia selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p;11.0; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

In another aspect, the biomarker further comprises microRNAs that are underexpressed in colorectal neoplasia and are selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

In another aspect, the biomarker further comprises microRNAs that are overexpressed in colorectal neoplasia and are selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p:11.0; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

The skilled artisan will recognize that most often a biosignature (assay) will include the combination of both over and underexpressed microRNAs. As such, the present invention also includes in on aspect the combination of both over and underexpressed microRNAs from respective microRNAs. In another aspect, the biological samples are selected from the group consisting of one or more biological fluids, a plasma sample, a serum sample, a blood sample, a tissue sample, or a fecal sample. In another aspect, the method is capable of detecting early CRC (I-II) as accurately as advanced CRC (stage II-III), right-sided tumors and left-sided lesions. In another aspect, the overall expression pattern or level of 4, 5, 6, 7, 8, 9, 10, 12, 15, 20, 25, 30, 35, 40, 45, 50, 55 or 60 microRNAs selected from Tables 2, 3, 4, and 5, wherein the microRNAs increase the specificity of the determination, diagnosis or detection of colorectal neoplasia.

Yet another embodiment includes a kit for a diagnosis of colorectal neoplasia comprising: biomarker detecting reagents for determining a differential expression level of miR19a and miR19b, or miR19a and miR19b and miR15b **microRNAs**, wherein overexpression of a combination of miR19a and miR19b, or miR19a and miR19b and miR15b is indicative of colorectal neoplasia, wherein a confidence interval for colorectal cancer is 90% or greater. In one aspect, the kit further comprises reagents for the detection and analysis of at least one of miR18a, miR29a, or miR335. In one aspect, the kit further comprises reagents for the detection and analysis of at least one of miR29a, miR92a or miR141. In another aspect, the kit further comprises instructions for use in diagnosing risk for colorectal neoplasia, wherein the instruction comprise step-by-step directions to compare the expression level of the microRNAs, when measuring the expression of a sample obtained from a subject suspected of having colorectal neoplasia with the expression level of a sample obtained from a normal subject, wherein the normal subject is a healthy subject not suffering from colorectal neoplasia. In another aspect, the kit further comprises tools, vessels and reagents necessary to obtain samples from a subject selected from the group consisting of one or more biological fluids, a plasma sample, a serum sample, a blood sample, a tissue sample, or a fecal sample. In another aspect, the kit further comprises reagents for the analysis of microRNAs that are underexpressed in colorectal neoplasia and are selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

In another aspect, the kit further comprises reagents for the analysis of microRNAs that are overexpressed in colorectal neoplasia and are selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p:11.0; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

In another aspect, the kit further comprises reagents for the detection and analysis of expression pattern or level of expression for 4, 5, 6, 7, 8, 9, 10, 12, 15, 20, 25, 30, 35, 40, 45, 50, 55 or 60 microRNAs is determined to diagnose or detect colorectal neoplasia selected from the microRNAs of Tables 2, 3, 4 and 5.

Yet another embodiment includes a method for selecting a cancer therapy for a patient diagnosed with colorectal neoplasia, the method comprising: obtaining a sample from a subject having a colorectal neoplasia; and determining the level of expression level of miR18a, miR19a, miR19b, miR15b, miR29a and miR335 as compared to the level of expression of a biological sample of a normal subject, wherein the normal subject is a healthy subject not suffering from colorectal neoplasia, wherein overexpression of the microRNAs is indicative of colorectal cancer; and selecting the cancer therapy based on the determination of the colorectal neoplasia in the patient.

Another embodiment includes a method of performing a clinical trial to evaluate a candidate drug believed to be useful in treating a disease state, the method comprising: (a) measuring the level of microRNAs obtained from a set of patients, wherein the microRNAs are selected from one or more microRNAs selected from miR19a and miR19b, or miR19a and miR19b and miR15b microRNAs (b) administering a candidate drug to a first subset of the patients, and a placebo to a second subset of the patients; a comparator drug to a second subset of the patients; or a drug combination of the candidate drug and another active agent to a second subset of patients; (c) repeating step (a) after the administration of the candidate drug or the placebo, the comparator drug or the drug combination; and (d) determining if the candidate drug reduces the number of colorectal neoplastic cells that have a change in the expression of the microRNAs that is statistically significant as compared to any change occurring in the second subset of patients, wherein a statistically significant reduction indicates that the candidate drug is useful in treating said disease state.

Yet another embodiment of the present invention includes a method for diagnosing or detecting colorectal neoplasia in a human subject comprising the steps of: identifying the human subject suffering from or suspected of suffering from colorectal neoplasia; obtaining one or more biological samples from the subject, wherein the biological samples are selected from of one or more biological fluids, a plasma sample, a serum sample, a blood sample, a tissue sample, or a fecal sample; measuring an overall expression pattern or level of miR15b alone or incombination with miR18a, miR19a, miR19b, miR29a and miR335; and comparing the overall expression pattern of the one or more microRNAs from the biological sample of the subject suspected of suffering from colorectal neoplasia with the overall expression pattern of the one or more microRNAs from a biological sample of a normal subject, wherein the normal subject is a healthy subject not suffering from colorectal neoplasia, wherein overexpression of microRNAs: miR18a, miR19a, miR19b, miR15b, miR29a and miR335, is indicative of colorectal cancer.

Yet another embodiment of the present invention includes a method for diagnosing or detecting colorectal neoplasia in a human subject comprising the steps of: identifying the human subject suffering from or suspected of suffering from colorectal neoplasia; obtaining one or more biological samples from the subject, wherein the biological samples are selected from of one or more biological fluids, a plasma sample, a serum sample, a blood sample, a tissue sample, or a fecal sample; measuring an overall expression pattern or level of one or more microRNAs selected from:

| TOP 60miRNAs (LIMMA) | AUC | CI low | CI high | cutoff | S | Sp |
|---|---|---|---|---|---|---|
| hsa-miR-636 | 0.8079 | 0.6885 | 0.9273 | 0.6074 | 0.8537 | 0.6500 |
| hsa-miR-876-3p | 0.8402 | 0.7296 | 0.9509 | 0.6403 | 0.8537 | 0.7000 |
| hsa-miR-1537 | 0.8524 | 0.9464 | 0.9780 | 0.699 | 0.8293 | 0.8000 |
| hsa-miR-630 | 0.8256 | 0.7218 | 0.9294 | 0.6619 | 0.7561 | 0.7500 |
| hsa-miR-380* | 0.8244 | 0.7269 | 0.9780 | 0.7065 | 0.7317 | 0.8000 |
| hsa-miR-338-5p | 0.8439 | 0.7296 | 0.9581 | 0.7118 | 0.7317 | 0.8500 |
| hsa-miR-573 | 0.8354 | 0.7243 | 0.9464 | 0.6829 | 0.8500 | 0.7592 |
| hsa-miR-182* | 0.8622 | 0.7687 | 0.9557 | 0.5313 | 0.8780 | 0.7000 |
| hsa-miR-518c* | 0.8610 | 0.7471 | 0.9748 | 0.5781 | 0.9024 | 0.8000 |
| hsa-miR-187* | 0.8537 | 0.7435 | 0.9638 | 0.7095 | 0.7561 | 0.8500 |
| hsa-miR-1233 | 0.8707 | 0.7587 | 0.9827 | 0.5828 | 0.9268 | 0.8000 |
| hsa-miR-449b | 0.8329 | 0.7146 | 0.9512 | 0.6777 | 0.8049 | 0.8500 |
| hsa-miR-1204 | 0.8622 | 0.7545 | 0.9699 | 0.6715 | 0.8293 | 0.8500 |
| hsa-miR-518d-3p | 0.8512 | 0.7389 | 0.9635 | 0.5514 | 0.9024 | 0.7500 |
| hsa-miR-1290 | 0.8439 | 0.7338 | 0.9540 | 0.7042 | 0.7805 | 0.8000 |
| hsa-miR-144:9.1 | 0.8524 | 0.7497 | 0.9552 | 0.7249 | 0.7561 | 0.8000 |
| hsa-miR-105 | 0.8866 | 0.7972 | 0.9760 | 0.6281 | 0.8780 | 0.8000 |
| hsa-miR-298 | 0.8805 | 0.7846 | 0.9764 | 0.6964 | 0.8049 | 0.8500 |
| hsa-miR-491-5p | 0.8610 | 0.7412 | 0.9807 | 0.6511 | 0.8780 | 0.8000 |
| hsa-miR-576-3p | 0.8866 | 0.7900 | 0.9830 | 0.635 | 0.8537 | 0.8000 |
| hsa-miR-590-3p | 0.8329 | 0.7122 | 0.9536 | 0.6806 | 0.7805 | 0.8000 |
| hsa-miR-1257 | 0.8451 | 0.7254 | 0.9649 | 0.6496 | 0.7805 | 0.8000 |
| hsa-miR-1225-3p | 0.8683 | 0.7550 | 0.9815 | 0.6726 | 0.8293 | 0.8500 |
| hsa-miR-127-3p | 0.8683 | 0.7648 | 0.9718 | 0.5883 | 0.9024 | 0.7500 |
| hsa-miR-936 | 0.8744 | 0.7743 | 0.9745 | 0.5981 | 0.8780 | 0.8000 |
| hsa-miR-379 | 0.8732 | 0.7839 | 0.9624 | 0.5632 | 0.9268 | 0.7000 |
| hsa-miR-664* | 0.8171 | 0.6895 | 0.9446 | 0.6124 | 0.9024 | 0.7000 |
| hsa-miR-548j | 0.8232 | 0.7127 | 0.9337 | 0.5905 | 0.8537 | 0.7000 |
| hsa-miR-130b* | 0.8518 | 0.7534 | 0.9502 | 0.7281 | 0.7805 | 0.7500 |
| hsa-miR-515-3p | 0.8659 | 0.7779 | 0.9538 | 0.5678 | 0.8537 | 0.7000 |
| hsa-miR-302b | 0.8280 | 0.7084 | 0.9477 | 0.5507 | 0.9024 | 0.7000 |
| hsa-miR-125a-5p | 0.8354 | 0.7242 | 0.9465 | 0.7217 | 0.7317 | 0.8500 |
| hsa-miR-424 | 0.8463 | 0.7439 | 0.9488 | 0.6183 | 0.8537 | 0.7000 |
| hsa-miR-125b | 0.8488 | 0.7417 | 0.9558 | 0.7011 | 0.8049 | 0.8000 |
| hsa-miR-100 | 0.8463 | 0.7328 | 0.9599 | 0.6536 | 0.8780 | 0.8000 |
| hsa-miR-768-3p:11.0 | 0.8110 | 0.6945 | 0.9275 | 0.6736 | 0.7561 | 0.7500 |
| hsa-miR-24 | 0.8317 | 0.7142 | 0.9493 | 0.6589 | 0.7805 | 0.7500 |
| hsa-miR-23a | 0.8659 | 0.7626 | 0.9690 | 0.5803 | 0.9268 | 0.7000 |
| hsa-miR-1274b | 0.8390 | 0.7303 | 0.9477 | 0.7056 | 0.7561 | 0.8000 |
| hsa-miR-27a | 0.8049 | 0.6821 | 0.9276 | 0.7093 | 0.7561 | 0.7500 |
| hsa-miR-26b | 0.8220 | 0.7122 | 0.9317 | 0.714 | 0.7561 | 0.7500 |
| hsa-miR-30d | 0.8311 | 0.7199 | 0.9422 | 0.6859 | 0.7805 | 0.8000 |
| hsa-miR-520h,hsa-miR-520g | 0.8427 | 0.7403 | 0.9451 | 0.7064 | 0.7561 | 0.8500 |
| hsa-miR-302a | 0.9024 | 0.8114 | 0.9935 | 0.6163 | 0.9268 | 0.8000 |
| hsa-miR-518c | 0.8610 | 0.7159 | 0.9402 | 0.5781 | 0.9024 | 0.8000 |
| hsa-miR-335 | 0.8061 | 0.6837 | 0.9285 | 0.6744 | 0.7805 | 0.7000 |
| hsa-miR-29a | 0.8573 | 0.7427 | 0.9719 | 0.6418 | 0.8293 | 0.8000 |
| hsa-miR-152 | 0.8329 | 0.7287 | 0.9372 | 0.716 | 0.7561 | 0.7500 |
| hsa-miR-191 | 0.8463 | 0.7387 | 0.9540 | 0.6702 | 0.8293 | 0.7500 |
| hsa-miR-17 | 0.8329 | 0.7164 | 0.9495 | 0.6218 | 0.6218 | 0.8000 |
| hsa-miR-19b | 0.8238 | 0.7006 | 0.9470 | 0.6582 | 0.8293 | 0.7500 |
| hsa-miR-30a | 0.8720 | 0.7641 | 0.9798 | 0.6823 | 0.8049 | 0.8500 |
| hsa-miR-151-5p | 0.8280 | 0.7062 | 0.9499 | 0.6133 | 0.8537 | 0.7500 |
| hsa-miR-92a | 0.8598 | 0.7634 | 0.9561 | 0.5437 | 0.9268 | 0.6500 |
| hsa-miR-25 | 0.8549 | 0.7572 | 0.9525 | 0.7136 | 0.7561 | 0.8000 |
| hsa-miR-15b | 0.8329 | 0.7215 | 0.9443 | 0.6642 | 0.8049 | 0.7000 |
| hsa-miR-15a | 0.8585 | 0.7564 | 0.9607 | 0.6298 | 0.8537 | 0.7500 |
| hsa-miR-30e* | 0.8707 | 0.7810 | 0.9605 | 0.7165 | 0.7805 | 0.8500 |
| hsa-miR-132* | 0.8463 | 0.7280 | 0.9647 | 0.5986 | 0.9024 | 0.8000 |
| hsa-miR-921 | 0.8476 | 0.7263 | 0.9689 | 0.6769 | 0.8537 | 0.8500 |

wherein ROC are the area under curve (AUC) parameters, CI is a 95% confidence interval, S is the sensitivity, and Sp is the specificity; and comparing the overall expression pattern of the one or more microRNAs obtained from the biological sample of the subject suspected of suffering from colorectal neoplasia with the overall expression pattern of the one or more microRNAs from a biological sample of a normal subject, wherein the normal subject is a healthy subject not suffering from colorectal neoplasia, wherein a change in the overall expression pattern of the one or more microRNAs in the biological sample of the subject is indicative of colorectal neoplasia. In one aspect, the microRNAs are underexpressed in colorectal neoplasia and are selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

In another aspect, the microRNAs are overexpressed in colorectal neoplasia and are selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p:110; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

In another aspect, the expression level of the one or more microRNAs is measured by microarray expression profiling, PCR, reverse transcriptase PCR, reverse transcriptase real-time PCR, quantitative real-time PCR, end-point PCR, multiplex end-point PCR, cold PCR, ice-cold PCR, mass spectrometry, in situ hybridization (ISH), multiplex in situ hybridization, or nucleic acid sequencing. In another aspect, the method is used for treating a patient at risk or suffering from colorectal neoplasia, selecting an anti-neoplastic agent therapy (e.g., nucleic acid crosslinking agents, small molecules, biologics such as monoclonal antibodies with or without cell killing payloads, both targeted and untargeted) for a patient at risk or suffering from colorectal neoplasia, stratifying a patient to a subgroup of colorectal neoplasia or for a colorectal neoplasia therapy clinical trial, determining resistance or responsiveness to a colorectal neoplasia therapeutic regimen, developing a kit for diagnosis of colorectal neoplasia or any combinations thereof. In another aspect, the overall expression pattern or level of 2, 3, 4, 5, 6, 7, 8, 9, 10, 12, 15, 20, 25, 30, 35, 40, 45, 50, 55 or 60 microRNAs is determined to diagnose or detect colorectal neoplasia.

### BRIEF DESCRIPTION OF THE DRAWINGS

For a more complete understanding of the features and advantages of the present invention, reference is now made to the detailed description of the invention along with the accompanying figures and in which:
Figs. 1A and 1B show the differential miRNA expression by microarrays between patients with CRC and controls from set 1 (Figure 1A), and between patients with AA and controls (Figure 1B). The heatmap shows the 50 significantly deregulated miRNAs with the highest FC. Red pixels correspond to an increased abundance of miRNA in the indicated plasma sample, whereas green pixels indicate decreased miRNA levels.
Fig. 2 is a Between Group Analysis (BGA) plot showing sample clustering based on miRNA expression profiling. Healthy controls (C); patients with colorectal cancer (CRC); patients with advanced adenomas (AA).
Fig. 3 shows box-plots showing plasma miRNA expression in the CRC set 2 determined by qRT-PCR. Expression levels of miRNAs are normalized to miR16 and represented as -dCt values. The lines inside the boxes denote the medians. The boxes mark the interval between the 25th and 75th percentiles.
Figs. 4A and 4B are Receiver Operating Characteristics (ROC) analysis for the two-plasma miRNA signature: miR19a+miR19b (Figure 4A) and three-plasma miRNA signature: miR19a+miR19b+miR15b (Figure 4B) according to the results obtained from microarray profiling in CRC set 1 and qRT-PCR data in CRC set 2.

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

Abbreviations: advanced adenomas, AA; area under the ROC curve, AUC; between group analysis, BGA; colorectal cancer, CRC; fold-change, FC; linear models for microarray data, LIMMA; microRNA, miRNA; receiver operating characteristics curve, ROC curve.

As used herein, the term "colorectal cancer" and "colorectal neoplasia" includes the well-accepted medical definition that defines colorectal cancer as a medical condition characterized by cancer of cells of the intestinal tract below the small intestine (i.e., the large intestine (colon), including the cecum, ascending colon, transverse colon, descending colon, sigmoid colon, and rectum) and includes pre-cancer (also referred to herein as advanced adenomas), early-stage, and late-stage cancer. Additionally, as used herein, the term "colorectal cancer" also further includes medical conditions that are characterized by cancer of cells of the duodenum and small intestine (jejunum and ileum).

As used herein, the term "tissue sample" (the term "tissue" is used interchangeably with the term "tissue sample") refers to include any material composed of one or more cells, either individual or in complex with any matrix or in association with any chemical. The definition shall include any biological or organic material and any cellular subportion, product or by-product thereof. The definition of "tissue sample" should be understood to include without limitation sperm, eggs, embryos and blood components. Also included within the definition of "tissue" for purposes of this invention are certain defined acellular structures such as dermal layers of skin that have a cellular origin but are no longer characterized as cellular. The term "stool" as used herein is a clinical term that refers to feces excreted by humans.

As used herein, the term "gene" refers to a functional protein, polypeptide or peptide-encoding unit. As will be understood by those in the art, this functional term includes genomic sequences, cDNA sequences, or fragments or combinations thereof, as well as gene products, including those that may have been altered by the hand of man. Purified genes, nucleic acids, protein and the like are used to refer to these entities when identified and separated from at least one contaminating nucleic acid or protein with which it is ordinarily associated. The term "allele" or "allelic form" refers to an alternative version of a gene encoding the same functional protein but containing differences in nucleotide sequence relative to another version of the same gene.

As used herein, the term "microRNA" ("miRNA" or "miR") refers to an RNA (or RNA analog) comprising the product of an endogenous, non-coding gene whose precursor RNA transcripts can form small stem-loops from which mature "miRNAs" are cleaved by, e.g., Dicer. "miRNAs" are encoded in genes distinct from the mRNAs whose expression they control.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

The term "biomarker" as used herein in various embodiments refers to a specific biochemical in the body that has a particular molecular feature to make it useful for diagnosing and measuring the progress of disease or the effects of treatment. For example, common metabolites or biomarkers found in a person's breath, and the respective diagnostic condition of the person providing such metabolite include, but are not limited to, acetaldehyde (source: ethanol, X-threonine; diagnosis: intoxication), acetone (source: acetoacetate; diagnosis: diet/diabetes), ammonia (source: deamination of amino acids; diagnosis: uremia and liver disease), CO (carbon monoxide) (source: CH₂Cl₂, elevated % COHb; diagnosis: indoor air pollution), chloroform (source: halogenated compounds), dichlorobenzene (source: halogenated compounds), diethylamine (source: choline; diagnosis: intestinal bacterial overgrowth), H (hydrogen) (source: intestines; diagnosis: lactose intolerance), isoprene (source: fatty acid; diagnosis: metabolic stress), methanethiol (source: methionine; diagnosis: intestinal bacterial overgrowth), methylethylketone (source: fatty acid; diagnosis: indoor air pollution/diet), O-toluidine (source: carcinoma metabolite; diagnosis: bronchogenic carcinoma), pentane sulfides and sulfides (source: lipid peroxidation; diagnosis: myocardial infarction), H2S (source: metabolism; diagnosis: periodontal disease/ovulation), MeS (source: metabolism; diagnosis: cirrhosis), and Me2S (source: infection; diagnosis: trench mouth).

The term "statistically significant" differences between the groups studied, relates to condition when using the appropriate statistical analysis (e.g. Chi-square test, t-test) the probability of the groups being the same is less than 5%, e.g. p<0.05. In other words, the probability of obtaining the same results on a completely random basis is less than 5 out of 100 attempts.

The term "kit" or "testing kit" denotes combinations of reagents and adjuvants required for an analysis. Although a test kit consists in most cases of several units, one-piece analysis elements are also available, which must likewise be regarded as testing kits.

The term "polymerase chain reaction" (PCR) as used herein refers to the method of K.B. Mullis, U.S. Patent Nos. 4,683,195, 4,683,202, and 4,965,188, hereby incorporated by reference, which describes a method for increasing the concentration of a segment of a target sequence in a mixture of genomic DNA without cloning or purification. This process for amplifying the target sequence consists of introducing a large excess of two oligonucleotide primers to the DNA mixture containing the desired target sequence, followed by a precise sequence of thermal cycling in the presence of a DNA polymerase. The two primers are complementary to their respective strands of the double stranded target sequence. To effect amplification, the mixture is denatured and the primers then annealed to their complementary sequences within the target molecule. Following annealing, the primers are extended with a polymerase so as to form a new pair of complementary strands. The steps of denaturation, primer annealing and polymerase extension can be repeated many times (i.e., denaturation, annealing and extension constitute one "cycle"; there can be numerous "cycles") to obtain a high concentration of an amplified segment of the desired target sequence. The length of the amplified segment of the desired target sequence is determined by the relative positions of the primers with respect to each other, and therefore, this length is a controllable parameter. By virtue of the repeating aspect of the process, the method is referred to as the "polymerase chain reaction" (hereinafter PCR).

As used herein, the one or more microRNAs may be measured by microarray expression profiling, PCR, reverse transcriptase PCR, reverse transcriptase real-time PCR, quantitative real-time PCR, end-point PCR, multiplex end-point PCR, ice-cold PCR, mass spectrometry, in situ hybridization (ISH), multiplex in situ hybridization, or nucleic acid sequencing. However, other techniques for determining expression of microRNAs may be used such as surface plasmon resonance, fluorescence resonance effects (transfer, quenching and variants thereof), or the next generation of any of the above listed techniques and combinations thereof, all of which are within the scope of the present invention. The overall level of expression of the one or more microRNAs can be used to increase the sensitivity and quality of the determination of the presence of the colorectal neoplasia. For example, it is typical that an increase in the sensitivity is accompanied by an increase in the number of microRNAs measured, e.g., as more microRNAs are measured (e.g., 2 versus 8 or 15 versus 30) there is a concomitant increase in the quality of the determination as is well-known to skilled artisans in the area of expression levels. The skilled artisan will recognize that most often a biosignature (assay) will include the combination of both over and underexpressed microRNAs. As such, the present invention also includes in on aspect the combination of both over and underexpressed microRNAs from respective microRNAs.

The present invention may be used for the diagnosis and treatment of patients, which includes or can be extended to prognosis, treatment guidance, monitoring response to treatment, use in clinical trials, research and combinations thereof. The skilled artisan will recognize that the detection of the microRNAs identified herein may be used for any of these uses.

MicroRNAs (miRNAs) are evolutionarily conserved, endogenous, small non-coding RNA molecules of 20-22 nucleotides that function as regulators of gene expression. Recent evidences have shown that miRNAs regulate diverse crucial cell processes such as development, differentiation, proliferation and apoptosis. They are thought to play an important role in initiation and progression of human cancer, acting as oncogenes or tumor suppressors[1].

Changes in expression profiles of miRNAs in various tissues have been observed in a variety of human pathologies including cancer. To date, every type of tumor analyzed by miRNA tissue profiling has shown significantly different miRNA profiles compared to normal cells from the same tissue[2-4]. Moreover, some recent reports have demonstrated that miRNAs are present in the serum and plasma of humans and other animals[5-7]. Circulating levels of miRNAs are quite stable, reproducible, and consistent among individuals of the same species[5]. Therefore, expression profiling of circulating miRNAs shows great promise as a novel non-invasive strategy for diagnosis of cancer and other diseases.

Colorectal cancer (CRC) is the second most common cancer in Western countries, and represents the second leading cause of cancer-related death[8]. Fortunately, there is evidence that screening of average-risk individuals can result in mortality and incidence reduction by early cancer detection and removal of cancer precursor lesions[9]. Indeed, the goal of screening programs is the detection of early-stage CRC and advanced adenomas (AA) which are premalignant lesions associated with a high risk of progression to an invasive lesion.

Currently, there is not optimal and universally accepted strategy for CRC screening [10,11] and fecal-based tests are hampered by their limited sensitivity, colonoscopy constitutes an invasive approach[12]. Therefore, new approaches that can complement and improve on current strategies are urgently needed. In that sense, previous studies have found that some miRNAs are increased in plasma from patients with CRC, suggesting a potential role as non-invasive biomarkers(13-16). However, all of them were limited to the analysis of a small number of miRNAs. Accordingly, it is mandatory to further characterize plasma miRNA profiling by high-throughput techniques and evaluate its performance characteristics in detecting individuals harboring CRC and/or cancer precursor lesions. In the present study, we performed plasma miRNA profiling by microarrays in a set of patients with CRC or AA, and healthy individuals, identifying a group of miRNAs able to detect patients with colorectal neoplasia with high discriminative capacity. Validation in an independent cohort of individuals and using a different technology allows us to confirm 6 plasma miRNAs as very promising biomarkers for non-invasive diagnosis of CRC. Unfortunately, the discriminative capacity of these miRNAs was limited in detecting cancer precursor lesions.

Circulating miRNAs show great promise as novel biomarkers for diagnosis of cancer and other diseases. New non-invasive approaches that can complement and improve on current strategies for colorectal cancer (CRC) screening are urgently needed. Genome-wide plasma miRNA expression profiling was performed by microarrays in a set of individuals (n=61) including patients with CRC or with pre-malignant lesions such as advanced colorectal adenomas (AA), and healthy subjects. Real-time qRT-PCR was used to validate the expression of selected miRNAs in an independent cohort of patients from another hospital (n=135). Patients with CRC or AA showed significantly different plasma miRNA expression profiles compared to controls. A group of 13 miRNAs was selected to be validated in an independent cohort of patients, and 6 out of them were confirmed to be significantly overexpressed in the CRC group, showing a high discriminative accuracy. One of these 6 miRNAs was confirmed to also be significantly overexpressed in patients with AA, with a moderate discriminative capacity.

A total of 273 subjects from two hospitals (Hospital Clinic of Barcelona, Catalonia, Spain and Hospital of Donostia, Gipuzkoa, Spain) were prospectively included in this study. Of them, 77 were excluded because they met any of the following criteria: clinical diagnosis of familial adenomatous polyposis or Lynch syndrome, presence of more than 10 colorectal adenomas, diagnosis of cancer at other sites at the time of selection, presenting inflammatory bowel disease, undergoing chemotherapy or radiation therapy at the time of blood sampling, incomplete bowel examination, inadequate bowel preparation at diagnostic colonoscopy, or presence of haemolysis in plasma samples. Finally, 196 individuals were included: 123 patients newly diagnosed with sporadic colorectal neoplasia (63 with CRC and 40 with AA) and 73 healthy individuals without personal history of any cancer and with a recent colonoscopy confirming the lack of colorectal neoplastic lesions. Patients with AA were those with adenomas having a size of at least 10 mm or histologically having high grade dysplasia or ≥20% villous component. These individuals were divided into two different and unrelated sets: set 1, 61 subjects from Hospital Clinic of Barcelona, which were employed to perform genome-wide plasma microRNA expression profiling; and set 2,135 subjects from Hospital of Donostia, which were recruited to further validate the results obtained in set 1. The characteristics of participants are shown in Table 1. Blood samples were collected prior to endoscopy or surgery in all individuals.

**Table 1. Patients characteristics.**

| | **Set 1 (microarray)** | | **Set 2 (qRT-PCR)** | |
|---|---|---|---|---|
| | Control (n=20) | Neoplasm (n=41) | Control (n=53) | Neoplasm (n=82) |
| Mean age (SD) | 60.6 (12.5) | 72.5 (9.7) | 62.1 (3.5) | 62.8 (6.3) |
| Gender -no. | | | | |
| Male | 11 | 20 | 26 | 42 |
| Female | 9 | 21 | 27 | 40 |

| Colorectal cancer features | | | | |
|---|---|---|---|---|
| Patients -no. | - | 21 | - | 42 |
| TNM stage -no. | | | | |
| I | - | 4 | - | 8 |
| II | - | 8 | - | 13 |
| III | - | 6 | - | 16 |
| IV | - | 3 | - | 5 |
| Location -no. | | | | |
| Proximal | - | 10 | - | 14 |
| Distal | - | 11 | - | 28 |
| Adenoma features | | | | |
| Patients -no. | - | 20 | - | 40 |
| Size ≥1 cm -no. | - | 20 | - | 36 |
| Mean size (mm) | - | 22.4 | - | 11.5 |
| High-grade dysplasia - no. | - | 3 | - | 0 |
| Villous component -no. | - | 10 | - | 23 |
| Sessile morphology -no. | - | 10 | - | 5 |

The study was approved by the Institutional Ethics Committee of Hospital Clinic of Barcelona (approval date: 03/26/2009), and written informed consent was obtained from all participants in accordance with the Declaration of Helsinki.

RNA extraction from plasma samples. Twenty ml of whole blood from each participant were collected in EDTA tubes. Blood samples were placed at 4°C until plasma separation, and plasma was frozen within 6 hours of the blood draw. Briefly, samples were centrifuged at 1,600 x g for 10 min at 4°C to spin down blood cells, and plasma was transferred into new tubes, followed by further centrifugation at 16,000 x g for 10 minutes at 4°C to completely remove cellular components. Plasma was then aliquoted and stored at -80°C until use. Total RNA containing small RNAs was isolated from 550 µl of plasma using Trizol LS reagent (Invitrogen, Carlsbad, CA) and miRNeasy Mini Kit (Qiagen, Hilden, Germany), according to the manufacturer protocol with the following modifications. Trizol LS reagent was added to plasma samples in a volumetric ratio 2:1. After phase separation by chloroform addition and centrifugation, aqueous phase was separated into a new tube and one volume of Trizol LS was further added. After the second phase separation 1.5 volume of 100% ethanol was added to the aqueous phase and the mixture was loaded into a miRNeasy column, according to the manufacturer instructions. DNase treatment (Qiagen) was carried out to remove any contaminating DNA. The final elution volume was 30 µl. RNA concentration was quantified using NanoDrop 1000 (Nanodrop, Wilmington, DE) in all samples and it ranged from 3 to 35 ng/µl. The extraction process was repeated for each sample until obtaining enough RNA quantity for next steps.

Genome-wide plasma miRNA profiling by microarray. MiRNA expression profiling was performed in all samples from set 1 using the MicroRNA Expression Profiling Assay based on the SAM-Bead Array platform (Illumina, Inc. San Diego, CA). This microarray contains 1,146 probes, including 743 validated miRNAs, detecting around 97% of the annotated human miRNAs in the miRBase Sanger v.12.0 database. The miRNA microarray assay was performed using 200 ng of total RNA per sample. All steps were performed according to the manufacturer protocol, as previously described [13,14]. Data were extracted using BeadStudio data analysis software and transformed to the log base 2 scale. Microarray data from all samples were quantile-normalized using Lumi bioconductor package[15].

Analysis of miRNA expression by real-time qRT-PCR. MiRNA expression was analyzed by real-time qRT-PCR with a previous multiplex preamplification process. Briefly, 21 ng of plasma RNA was retrotranscribed with a mix of Megaplex RT Primers (Applied Biosystems Inc., Foster City, CA) and preamplified with Megaplex PreAmp Primers and TaqMan PreAmp MasterMix (Applied Biosystems Inc.) for 14 cycles. The expression of each miRNA was assessed by qPCR using TaqMan miRNA Assays (Applied Biosystems Inc.) in a Viia7 Real-Time PCR System (Applied Biosystems Inc.). Several putative housekeeping small nuclear RNAs were analyzed in order to determine the most suitable in our samples (RNU6B, miR16, miR423-5p, RNU48, miR544, miR103, miR525, miR451). MiR16 displayed the highest stability and abundance and, therefore, expression levels of miRNAs were normalized to miR16 as internal control in concordance with other publications [16,17]. Ct values were calculated from automatic threshold. No template controls showed no amplification. Three technical replicates were included for each point of qPCR. Relative expression levels of selected miRNAs were calculated for each sample as ΔCt values [ΔCt = Ct of target miRNA - Ct of internal control miRNA].

Statistical analysis. A Linear Models for Microarray Data (LIMMA) was used to identify miRNAs differentially expressed between groups. LIMMA uses linear models and empirical Bayes paired moderated t-statistics and F-statistics[18]. The most significant miRNAs using F-statistics were used to perform correspondence analysis as implemented in the between group analysis (BGA) function included in the made4 package[19]. This method is capable of visualizing high-dimensional data (such as multiple expression measurements) in a 2D graph in which the areas delimited by the ellipses represent 95% of the estimated binormal distribution of the sample scores on the first and second axes [20]. Venn Diagrams were made considering as a hit only miRNAs with an absolute fold change greater than 1.5 and a moderate p-value <0.05 (VennCounts and VennDiagram from LIMMA package). Quantitative variables were analyzed using the Student's test. A two-sided p value <0.05 was regarded as significant. Evaluation of predictability of individual miRNAs and different miRNAs combinations, adjusted by age and gender, were calculated using logistic regression (GLM binomial distribution). ROC analysis plots and derived cut-points, as well as overall discriminative accuracy parameters, were computed using DiagnosisMed R-package. The sensitivity and specificity were calculated from the optimum cut-point associated with the minimum distance between the ROC curve and upper left corner.

Genome-wide miRNA profiling in plasma samples from patients with colorectal cancer. Plasma miRNA expression discriminates patients with colorectal cancer from healthy individuals. To assess differential circulating miRNA expression profiling between patients with CRC and healthy individuals, miRNA microarray experiments were conducted on total RNA obtained from plasma samples of 21 patients with CRC and 20 healthy controls. To further investigate if altered miRNA expression patterns were found in patients with colorectal cancer precursor lesions, miRNA microarray experiments were also done on plasma RNA from 20 patients with advanced colorectal adenomas (AA).

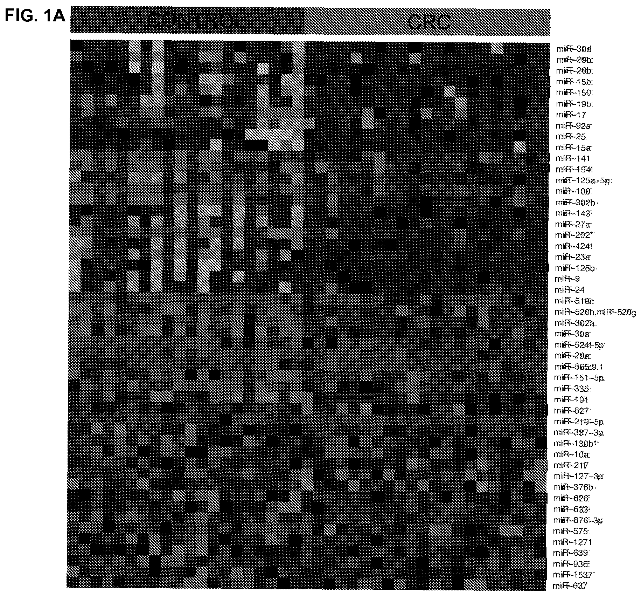

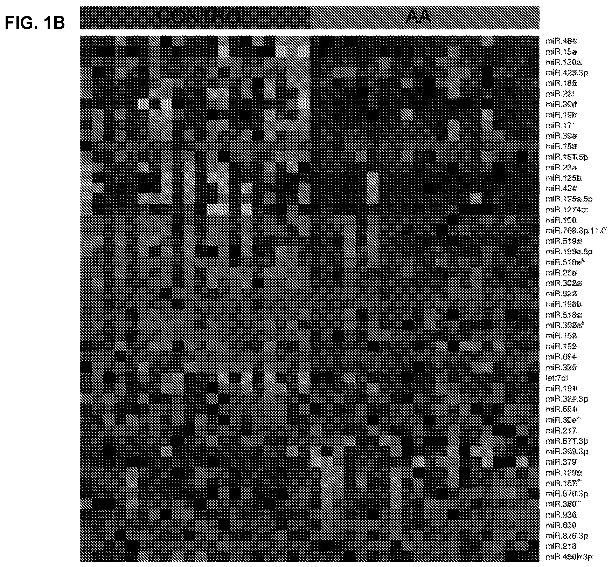

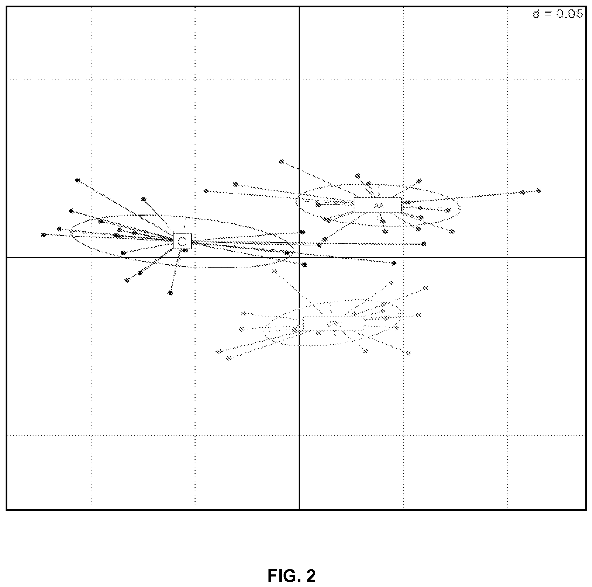

An initial comparative statistical analysis employing LIMMA yielded a total of 93 miRNAs significantly deregulated (p<0.05) in CRC patients in comparison to healthy individuals, and 125 miRNAs when AA patients were compared to healthy controls. All microarray data are available in GEO (accession number: GSE 25609). Figures 1A and 1B show heatmaps including the 50 miRNAs with the highest significant fold-change between CRC patients and controls (Figure 1A), and between AA patients and controls (Figure 1B). Fold-change differences and p-values, as well as the corresponding predictability parameters for these miRNAs in CRC or AA, are shown in Tables 2 -3 and 4-5, respectively. BGA graph was then performed to visually represent the proximity between patients harboring CRC or AA, and controls according to plasma miRNA expression. As depicted in Figure 2, patients with CRC or AA, and healthy individuals appeared as three clearly separated groups. The specificities of miRNA expression of each type of neoplastic lesion were also analyzed, i.e., CRC and AA, compared to control samples using Venn analysis (Figure 2). It was found that a subset of 21 and 28 miRNAs were exclusively and significantly up-regulated in patients with CRC and AA, respectively, whereas both type of neoplastic patients shared 24 significantly up-regulated miRNAs. Therefore, each colorectal neoplastic lesion has a particular miRNA expression profile but both of them also share an important number of deregulated miRNAs, which could allow identifying both lesions using a single test based on a common plasma miRNA signature.

**Table 2. Fold-change and p-value parameters for the top 50 deregulated miRNAs in CRC showing the highest fold-changes.**

| **Top 50 miRNAs** | **MIMAT #** | **FC** | **p** |
|---|---|---|---|
| hsa-miR-302a | MIMAT0000684 | 10,57 | 0,0002 |
| hsa-miR-30a | MIMAT0000087 | 9,43 | 0,0001 |
| hsa-miR-302b | MIMAT0000714 | 5,97 | 0,0042 |
| hsa-miR-565:9.1 | MI0003571 | 5,69 | 0,0010 |
| hsa-miR-191 | MIMAT0000440 | 5,32 | 0,0061 |
| hsa-miR-125b | MIMAT0000423 | 5,27 | 0,0022 |
| hsa-miR-100 | MIMAT0000098 | 4,69 | 0,0180 |
| hsa-miR-194 | MIMAT0000460 | 4,67 | 0,0160 |
| hsa-miR-27a | MIMAT0000084 | 4,53 | 0,0045 |
| hsa-miR-424 | MIMAT0001341 | 4,47 | 0,0055 |
| hsa-miR-125a-5p | MIMAT0000443 | 4,41 | 0,0060 |
| hsa-miR-335 | MIMAT0000765 | 4,16 | 0,0268 |
| hsa-miR-29a | MIMAT0000086 | 4,11 | 0,0237 |
| hsa-miR-219-5p | MIMAT0000276 | 4,00 | 0,0427 |
| hsa-miR-17 | MIMAT0000070 | 3,95 | 0,0386 |
| hsa-miR-520h/g | MIMAT0002867 | 3,92 | 0,0047 |
| hsa-miR-151-5p | MIMAT0004697 | 3,92 | 0,0131 |
| hsa-miR-524-5p | MIMAT0002849 | 3,82 | 0,0172 |
| hsa-miR-29b | MIMAT0000086 | 3,77 | 0,0340 |
| hsa-miR-202* | MIMAT0002810 | 3,51 | 0,0231 |
| hsa-miR-9 | MIMAT0000441 | 3,36 | 0,0074 |
| hsa-miR-150 | MIMAT0000451 | 3,30 | 0,0394 |
| hsa-miR-15b | MIMAT0000417 | 3,21 | 0,0019 |
| hsa-miR-518c | MIMAT0002848 | 3,20 | 0,0304 |
| hsa-miR-23a | MIMAT0000078 | 3,14 | 0,0003 |
| hsa-miR-19b | MIMAT0000074 | 3,03 | 0,0035 |
| hsa-miR-25 | MIMAT0004498 | 3,03 | 0,0060 |
| hsa-miR-15a | MIMAT0000068 | 3,02 | 0,0238 |
| hsa-miR-143 | MIMAT0004599 | 3,00 | 0,0069 |
| hsa-miR-141 | MIMAT0004598 | 2,77 | 0,0211 |
| hsa-miR-30d | MIMAT0000245 | 2,62 | 0,0315 |
| hsa-miR-627 | MIMAT0003296 | 2,62 | 0,0207 |
| hsa-miR-26b | MIMAT0000083 | 2,60 | 0,0059 |
| hsa-miR-24 | MIMAT0000080 | 2,46 | 0,0140 |
| hsa-miR-217 | MIMAT0000274 | 2,24 | 0,0278 |
| hsa-miR-92a | MIMAT0000092 | 1,83 | 0,0437 |
| hsa-miR-376b | MIMAT0002172 | -1,90 | 0,0281 |
| hsa-miR-637 | MIMAT0003307 | -1,90 | 0,0432 |
| hsa-miR-130b* | MIMAT0004680 | -1,95 | 0,0116 |
| hsa-miR-1537 | MIMAT0007399 | -1,97 | 0,0383 |
| hsa-miR-633 | MIMAT0003303 | -2,01 | 0,0448 |
| hsa-miR-10a | MIMAT0000253 | -2,12 | 0,0337 |
| hsa-miR-127-3p | MIMAT0000446 | -2,23 | 0,0072 |
| hsa-miR-337-3p | MIMAT0000754 | -2,23 | 0,0319 |
| hsa-miR-575 | MIMAT0003240 | -2,24 | 0,0257 |
| hsa-miR-936 | MIMAT0004979 | -2,25 | 0,0084 |
| hsa-miR-626 | MIMAT0003295 | -2,31 | 0,0361 |
| hsa-miR-1271 | MIMAT0005796 | -2,38 | 0,0315 |
| hsa-miR-876-3p | MIMAT0004925 | -2,65 | 0,0023 |
| hsa-miR-639 | MIMAT0003309 | -2,90 | 0,0222 |

**Table 3. Predictabilility of each individual miRNA among the top 50 deregulated miRNAs in CRC. ROC curve parameters (area under curve (AUC) and 95% confidence interval (CI), and sensitivity (S) and specificity (Sp) corresponding to an optimal cut-point are shown.**

| **TOP 50 miRNAs** | **AUC** | **CI low** | **CI high** | **cut-point** | **S** | **Sp** |
|---|---|---|---|---|---|---|
| hsa-miR-302a | 0,9143 | 0,8233 | 1,0053 | 0,5195 | 81 | 90 |
| hsa-miR-30a | 0,9048 | 0,7992 | 1,0103 | 0,5151 | 86 | 85 |
| hsa-miR-302b | 0,8524 | 0,7327 | 0,9720 | 0,5333 | 81 | 80 |
| hsa-miR-565:9.1 | 0,8905 | 0,7861 | 0,9948 | 0,5262 | 81 | 85 |
| hsa-miR-191 | 0,8667 | 0,7557 | 0,9776 | 0,4503 | 90 | 75 |
| hsa-miR-125b | 0,8714 | 0,7429 | 1,0000 | 0,5443 | 86 | 85 |
| hsa-miR-100 | 0,8643 | 0,7381 | 0,9904 | 0,5230 | 86 | 90 |
| hsa-miR-194 | 0,8238 | 0,6885 | 0,9591 | 0,4942 | 86 | 75 |
| hsa-miR-27a | 0,8405 | 0,7092 | 0,9717 | 0,5899 | 86 | 80 |
| hsa-miR-424 | 0,8619 | 0,7514 | 0,9725 | 0,5888 | 76 | 80 |
| hsa-miR-125a-5p | 0,8500 | 0,7272 | 0,9728 | 0,5901 | 76 | 85 |
| hsa-miR-335 | 0,8310 | 0,6973 | 0,9646 | 0,5494 | 81 | 85 |
| hsa-miR-29a | 0,8667 | 0,7435 | 0,9898 | 0,5449 | 81 | 85 |
| hsa-miR-219-5p | 0,8238 | 0,6967 | 0,9509 | 0,5895 | 76 | 75 |
| hsa-miR-17 | 0,8429 | 0,7185 | 0,9672 | 0,4718 | 81 | 80 |
| hsa-miR-520h/g | 0,8786 | 0,7724 | 0,9847 | 0,5382 | 81 | 85 |
| hsa-miR-151-5p | 0,8476 | 0,7183 | 0,9770 | 0,4912 | 90 | 80 |
| hsa-miR-524-5p | 0,8333 | 0,7023 | 0,9643 | 0,5795 | 76 | 90 |
| hsa-miR-29b | 0,8381 | 0,7136 | 0,9626 | 0,4574 | 90 | 75 |
| hsa-miR-202^{*} | 0,8405 | 0,7121 | 0,9689 | 0,4558 | 90 | 75 |
| hsa-miR-9 | 0,8381 | 0,7138 | 0,9624 | 0,5730 | 81 | 80 |
| hsa-miR-150 | 0,8429 | 0,7184 | 0,9673 | 0,4537 | 86 | 75 |
| hsa-miR-15b | 0,8643 | 0,7507 | 0,9778 | 0,5775 | 81 | 80 |
| hsa-miR-518c | 0,8476 | 0,7216 | 0,9737 | 0,5840 | 81 | 85 |
| hsa-miR-23a | 0,8905 | 0,7861 | 0,9948 | 0,5799 | 81 | 80 |
| hsa-miR-19b | 0,8381 | 0,7016 | 0,9746 | 0,6538 | 81 | 80 |
| hsa-miR-25 | 0,8952 | 0,8028 | 0,9876 | 0,5533 | 81 | 80 |
| hsa-miR-15a | 0,8619 | 0,7493 | 0,9745 | 0,4686 | 81 | 75 |
| hsa-miR-143 | 0,8524 | 0,7333 | 0,9714 | 0,5989 | 76 | 85 |
| hsa-miR-141 | 0,8429 | 0,7082 | 0,9776 | 0,5084 | 86 | 85 |
| hsa-miR-30d | 0,8226 | 0,6918 | 0,9534 | 0,5176 | 76 | 80 |
| hsa-miR-627 | 0,8333 | 0,7083 | 0,9584 | 0,5961 | 71 | 85 |
| hsa-miR-26b | 0,8619 | 0,7505 | 0,9733 | 0,5455 | 90 | 70 |
| hsa-miR-24 | 0,8524 | 0,7292 | 0,9756 | 0,4210 | 90 | 75 |
| hsa-miR-217 | 0,8310 | 0,7035 | 0,9584 | 0,5650 | 67 | 80 |
| hsa-miR-92a | 0,8571 | 0,7451 | 0,9692 | 0,2972 | 95 | 65 |
| hsa-miR-376b | 0,8262 | 0,7004 | 0,9520 | 0,6884 | 62 | 90 |
| hsa-miR-637 | 0,8595 | 0,7435 | 0,9756 | 0,4911 | 86 | 75 |
| hsa-miR-130b* | 0,8762 | 0,7694 | 0,9830 | 0,6915 | 71 | 95 |
| hsa-miR-1537 | 0,8524 | 0,7198 | 0,9849 | 0,4172 | 90 | 75 |
| hsa-miR-633 | 0,8595 | 0,7439 | 0,9751 | 0,5986 | 76 | 85 |
| hsa-miR-10a | 0,8810 | 0,7764 | 0,9855 | 0,4161 | 86 | 75 |
| hsa-miR-127-3p | 0,8833 | 0,7744 | 0,9922 | 0,5676 | 81 | 85 |
| hsa-miR-337-3p | 0,8452 | 0,7222 | 0,9683 | 0,5727 | 71 | 80 |
| hsa-miR-575 | 0,8298 | 0,7015 | 0,9580 | 0,6407 | 76 | 85 |
| hsa-miR-936 | 0,8476 | 0,7249 | 0,9704 | 0,5919 | 71 | 90 |
| hsa-miR-626 | 0,8214 | 0,6928 | 0,9501 | 0,6718 | 71 | 80 |
| hsa-miR-1271 | 0,8190 | 0,6896 | 0,9485 | 0,6845 | 67 | 85 |
| hsa-miR-876-3p | 0,8476 | 0,7212 | 0,9741 | 0,4657 | 90 | 75 |
| hsa-miR-639 | 0,8071 | 0,6753 | 0,9390 | 0,7118 | 52 | 95 |

**Table 4. Fold-change and p-value parameters for the top 50 deregulated miRNAs in AA showing the highest fold-changes.**

| **Top 50 miRNAs** | **MIMAT #** | **FC** | **p value** |
|---|---|---|---|
| hsa-miR-302a | MIMAT0000684 | 10,37 | 0,0003 |
| hsa-miR-29a | MIMAT0000086 | 9,12 | 0,0001 |
| hsa-miR-152 | MIMAT0000438 | 6,29 | 0,0002 |
| hsa-miR-518e* | MIMAT0005450 | 6,12 | 0,0079 |
| hsa-miR-768-3p:11.0 | MI0005117 | 5,68 | 0,0048 |
| hsa-miR-335 | MIMAT0000765 | 5,67 | 0,0112 |
| hsa-miR-17 | MIMAT0000070 | 5,46 | 0,0069 |
| hsa-miR-30a | MIMAT0000087 | 4,91 | 0,0142 |
| hsa-miR-191 | MIMA T0000440 | 4,82 | 0,0162 |
| hsa-miR-100 | MIMAT0000098 | 4,55 | 0,0235 |
| hsa-miR-519d | MIMAT0002853 | 4,24 | 0,0277 |
| hsa-miR-199a-5p | MIMAT0000231 | 4,11 | 0,0222 |
| hsa-miR-125a-5p | MIMAT0000443 | 3,96 | 0,0219 |
| hsa-miR-424 | MIMAT0001341 | 3,95 | 0,0174 |
| hsa-miR-18a | MIMAT0000072 | 3,88 | 0,0133 |
| hsa-miR-193b | MIMAT0002819 | 3,80 | 0,0394 |
| hsa-miR-302a* | MIMAT0000683 | 3,76 | 0,0340 |
| hsa-miR-151-5p | MIMAT0004697 | 3,75 | 0,0150 |
| hsa-miR-125b | MIMAT0000423 | 3,75 | 0,0275 |
| hsa-miR-518c | MIMAT0002848 | 3,67 | 0,0159 |
| hsa-miR-130a | MIMAT0000425 | 3,59 | 0,0079 |
| hsa-miR-30d | MIMAT0000245 | 3,58 | 0,0025 |
| hsa-miR-22 | MIMAT0000077 | 3,27 | 0,0096 |
| hsa-miR-15a | MIMAT0000068 | 3,25 | 0,0133 |
| hsa-miR-192 | MIMAT0000222 | 3,22 | 0,0404 |
| hsa-miR-484 | MIMAT0002174 | 3,18 | 0,0067 |
| hsa-miR-522 | MIMAT0002868 | 2,93 | 0,0394 |
| hsa-miR-423-3p | MIMAT0001340 | 2,88 | 0,0367 |
| hsa-miR-217 | MIMAT0000274 | 2,79 | 0,0056 |
| hsa-miR-664 | MIMAT0005949 | 2,70 | 0,0252 |
| hsa-miR-30e* | MIMAT0000693 | 2,63 | 0,0001 |
| hsa-miR-185 | MIMAT0000455 | 2,59 | 0,0357 |
| hsa-miR-19b | MIMAT0000074 | 2,57 | 0,0321 |
| hsa-miR-1274b | MI0006427 | 2,55 | 0,0216 |
| hsa-miR-23a | MIMAT0000078 | 2,51 | 0,0047 |
| hsa-miR-324-3p | MIMAT0000762 | 2,48 | 0,0118 |
| hsa-let-7d | MIMAT0000065 | 2,47 | 0,0300 |
| hsa-miR-581 | MIMAT0003246 | 2,46 | 0,0081 |
| hsa-miR-450b-3p | MIMAT0004910 | -2,49 | 0,0268 |
| hsa-miR-187* | MIMAT0004561 | -2,59 | 0,0002 |
| hsa-miR-576-3p | MIMAT0004796 | -2,59 | 0,0001 |
| hsa-miR-1290 | MIMAT0005880 | -2,62 | 0,0005 |
| hsa-miR-380* | MIMAT0000734 | -2,66 | 0,0016 |
| hsa-miR-369-3p | MIMAT0000721 | -2,84 | 0,0190 |
| hsa-miR-876-3p | MIMAT0004925 | -3,09 | 0,0022 |
| hsa-miR-671-3p | MIMAT0004819 | -3,52 | 0,0008 |
| hsa-miR-936 | MIMAT0004979 | -3,55 | 0,0001 |
| hsa-miR-218 | MIMAT0000275 | -4,56 | 0,0122 |
| hsa-miR-379 | MIMAT0000733 | -5,71 | 0,0000 |
| hsa-miR-630 | MIMAT0003299 | -6,29 | 0,0010 |

**Table 5. Predictabilility of each individual miRNA among the top 50 deregulated miRNAs in AA. ROC curve parameters (area under curve (AUC) and 95% confidence interval (CI), and sensitivity (S) and specificity (Sp) corresponding to an optimal cut-point are shown.**

| **TOP 50 miRNAs** | **AUC** | **CI low** | **CI high** | **cut-point** | **S** | **Sp** |
|---|---|---|---|---|---|---|
| miR.302a | 0,9100 | 0,8170 | 1,0030 | 0,3571 | 95 | 80 |
| miR.29a | 0,8775 | 0,7656 | 0,9894 | 0,3763 | 90 | 80 |
| miR.152 | 0,875 | 0,7700 | 0,9800 | 0,5804 | 80 | 85 |
| miR.518e* | 0,7925 | 0,6551 | 0,9299 | 0,5243 | 65 | 70 |
| miR.768.3p.11.0 | 0,8175 | 0,6888 | 0,9462 | 0,4317 | 80 | 65 |
| miR.335 | 0,7925 | 0,6545 | 0,9305 | 0,5461 | 65 | 75 |
| miR.17 | 0,8525 | 0,7340 | 0,9710 | 0,4344 | 80 | 75 |
| miR.30a | 0,8350 | 0,7050 | 0,9650 | 0,4889 | 80 | 80 |
| miR.191 | 0,8200 | 0,6888 | 0,9512 | 0,5530 | 75 | 75 |
| miR.100 | 0,8300 | 0,6963 | 0,9637 | 0,5106 | 80 | 80 |
| miR.519d | 0,7925 | 0,6527 | 0,9323 | 0,4689 | 80 | 70 |
| miR.199a.5p | 0,8050 | 0,6711 | 0,9389 | 0,4913 | 75 | 70 |
| miR.125a.5p | 0,8225 | 0,6922 | 0,9528 | 0,3773 | 90 | 65 |
| miR.424 | 0,8350 | 0,7128 | 0,9572 | 0,4925 | 75 | 70 |
| miR.18a | 0,8400 | 0,7164 | 0,9636 | 0,5475 | 80 | 80 |
| miR.193b | 0,7875 | 0,6473 | 0,9277 | 0,5452 | 75 | 75 |
| miR.302a* | 0,8200 | 0,6850 | 0,9550 | 0,5003 | 80 | 80 |
| miR.151.5p | 0,7950 | 0,6564 | 0,9336 | 0,4711 | 75 | 70 |
| miR.125b | 0,8250 | 0,6988 | 0,9512 | 0,3842 | 80 | 70 |
| miR.518c | 0,8475 | 0,7273 | 0,9677 | 0,5524 | 75 | 70 |
| miR.130a | 0,8275 | 0,6900 | 0,9650 | 0,5786 | 80 | 85 |
| miR.30d | 0,8725 | 0,7659 | 0,9791 | 0,3596 | 85 | 70 |
| miR.22 | 0,8525 | 0,7310 | 0,9740 | 0,5737 | 75 | 80 |
| miR.15a | 0,8725 | 0,7654 | 0,9796 | 0,4755 | 80 | 75 |
| miR.192 | 0,7800 | 0,6386 | 0,9214 | 0,4435 | 80 | 65 |
| miR.484 | 0,8625 | 0,7492 | 0,9758 | 0,3974 | 90 | 70 |
| miR.522 | 0,7975 | 0,6613 | 0,9337 | 0,5762 | 70 | 75 |
| miR.423.3p | 0,8150 | 0,6780 | 0,9520 | 0,5520 | 70 | 85 |
| miR.217 | 0,8525 | 0,7257 | 0,9793 | 0,5673 | 75 | 95 |
| miR.664 | 0,8175 | 0,6715 | 0,9635 | 0,5403 | 80 | 80 |
| miR.30e* | 0,8775 | 0,7709 | 0,9841 | 0,5039 | 85 | 80 |
| miR.185 | 0,8275 | 0,6976 | 0,9574 | 0,6138 | 65 | 85 |
| miR.19b | 0,8087 | 0,6746 | 0,9429 | 0,4663 | 80 | 70 |
| miR.1274b | 0,8400 | 0,7189 | 0,9611 | 0,5231 | 70 | 75 |
| miR.23a | 0,8400 | 0,7195 | 0,9605 | 0,4723 | 85 | 70 |
| miR.324.3p | 0,8275 | 0,6976 | 0,9574 | 0,5407 | 80 | 70 |
| let.7d | 0,8025 | 0,6645 | 0,9405 | 0,5203 | 75 | 70 |
| miR.581 | 0,8525 | 0,7340 | 0,9710 | 0,6819 | 70 | 90 |
| miR.450b.3p | 0,7550 | 0,6059 | 0,9041 | 0,5207 | 65 | 70 |
| miR.187* | 0,9075 | 0,8125 | 1,0025 | 0,5139 | 85 | 85 |
| miR.576.3p | 0,9575 | 0,8941 | 1,0209 | 0,4191 | 90 | 90 |
| miR.1290 | 0,8750 | 0,7669 | 0,9831 | 0,4742 | 85 | 80 |
| miR.380* | 0,8475 | 0,7156 | 0,9794 | 0,5532 | 75 | 90 |
| miR.369.3p | 0,7925 | 0,6533 | 0,9317 | 0,5663 | 65 | 80 |
| miR.876.3p | 0,8325 | 0,7090 | 0,9560 | 0,6131 | 70 | 80 |
| miR.671.3p | 0,8550 | 0,7393 | 0,9707 | 0,5179 | 65 | 85 |
| miR.936 | 0,9225 | 0,8427 | 1,0023 | 0,5084 | 85 | 85 |
| miR.218 | 0,8000 | 0,6591 | 0,9409 | 0,4740 | 75 | 70 |
| miR.379 | 0,9325 | 0,8519 | 1,0131 | 0,5830 | 85 | 95 |
| miR.630 | 0,8375 | 0,7132 | 0,9618 | 0,4579 | 75 | 70 |

Validation of plasma miRNA expression by real-time qRT-PCR. Microarray based plasma miRNA expression results are technically reproducible. Initially, a real-time qRT-PCR was performed to confirm microarray results in 28 samples randomly selected from set 1 (19 patients with colorectal neoplasms and 9 healthy controls). For these studies, a total of 14 candidate miRNAs were selected. Twelve candidate miRNAs (miR17-5p, miR92a, miR19b, miR18a, miR29a, miR302a, miR23a, miR27a, miR24, miR335, miR424 and miR15b) were chosen for being present in the top 50 deregulated miRNA in CRC and/or AA and to have a log base 2 microarray intensity ≥8. Two additional miRNAs (miR19a, and miR20a) were also selected for being part of the miR17-92 cluster, one of the best characterized oncogenic miRNA clusters, although they did not satisfy the previous criteria. Overall, qRT-PCR and microarray results were correlated (data not shown) except for miR424 that did not show any amplification by qRT-PCR and, therefore, it was excluded from subsequent analysis.

Six plasma microRNAs were confirmed to be overexpressed in CRC patients from an independent cohort. Secondly, the 13 candidate miRNAs that showed adequate amplification in the previous phase were analyzed (miR92a, miR17-5p, miR18a, miR19a, miR19b, miR20a, miR15b, miR29a, miR302a, miR23a, miR27a, miR24 and miR335) in plasma of an independent set of 42 patients with CRC and 53 healthy controls, to validate our results by real-time qRT-PCR.

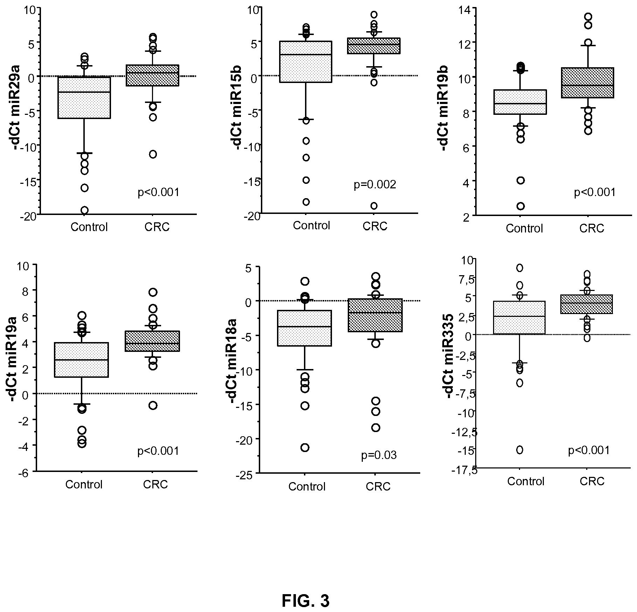

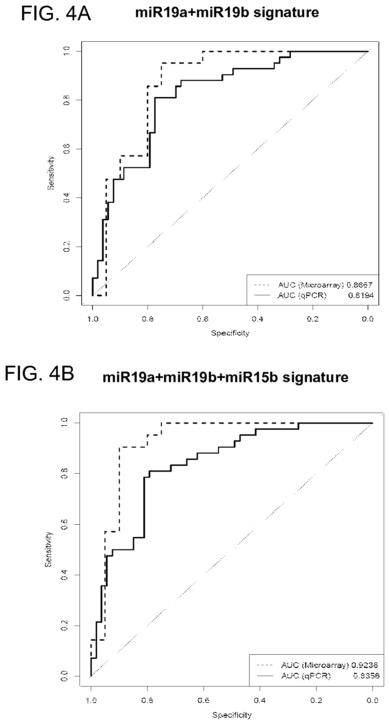

Interestingly, miR18a, miR19a, miR19b, miR15b, miR29a and miR335 were confirmed to be significantly up-regulated in patients with CRC (Figure 3). In addition, miR-24 was also overexpressed in this group of patients but without reaching statistical significance (p=0.08). Remarkably, validated miRNAs in this second set also demonstrated a high accuracy in discriminating CRC from healthy controls with areas under ROC curve (AUC) ranging from 0.8 (95% CI: 0.71-0.89) to 0.7 (95% CI: 0.59-0.80). Next, we sought to see if any combination of these miRNAs could improve the discriminative accuracy in detecting CRC with respect to each of them alone. Among the combinations showing the best discriminative capacity highlighted the signatures miR19a + miR19b, and miR19a + miR19b + miR15b (Table 6; Figure 4). Finally, we explored the predictive capacity of these signatures in early (TNM I-II) and advanced (TNM III-IV) CRC patients. As exposed in Table 2, both signatures showed a high discriminative accuracy in both early and advanced cases. Similarly, we examined whether these signatures were also able to detect right-sided tumours as accurately as left-sided ones, and it was the case for both (Table 2).

**Table 6. Predictability of the best plasma miRNA signatures in patients with CRC from set 2.**

| **SIGNATURES:** | **miR19a+miR19b** | **miR19a+miR19b+miR15b** |
|---|---|---|
| **All CRC (n=42)** | | |
| AUC (95% IC) | 0.82(0.73-0.90) | 0.84(0.76-0.92) |
| Sensitivity | 78.57 | 78.57 |
| Specificity | 77.36 | 79.25 |

| **TNM I/II (n=21)** | | |
|---|---|---|
| AUC (95% IC) | 0.85(0.75-0.96) | 0.87(0.71- 0.92) |
| Sensitivity | 71.43 | 80.95 |
| Specificity | 92.45 | 79.25 |

| **TNM III/IV (n=21)** | | |
|---|---|---|
| AUC (95% IC) | 0.81(0.71-0.92) | 0.81(0.70-0.92) |
| Sensitivity | 85.71 | 76.19 |
| Specificity | 71.70 | 77.36 |

| **Right-sided (n=14)** | | |
|---|---|---|
| AUC (95% IC) | 0.82(0.71-0.92) | 0.84(0.73-0.94) |
| Sensitivity | 85.71 | 85.71 |
| Specificity | 79.25 | 79.25 |

| **Left-sided (n=28)** | | |
|---|---|---|
| AUC (95% IC) | 0.8(0.71-0.90) | 0.83(0.73-0.92) |
| Sensitivity | 82.14 | 75.47 |
| Specificity | 78.57 | 79.25 |

ROC curve parameters (area under curve (AUC) and 95% confidence interval (CI) are shown for all CRC cases as well as for different tumor stages (I/II and III/IV) and locations (right and left, with respect to the splenic flexure)).

One plasma microRNA was confirmed to be increased in patients with advanced colorectal adenomas. In order to assess if any of the plasma miRNAs found overexpressed in both sets of CRC patients were also increased in patients harbouring cancer precursor lesions, which were analyzed by real-time qRT-PCR in plasma samples from an independent cohort of 40 patients with AA and 53 healthy controls. The miR18a was confirmed to be significantly overexpressed in this second set of AA patients in comparison to controls, as it was in the first set (Figure 1B). However, although this miRNA showed a good discriminative capacity in the first set of AA patients (AUC: 0.84, 95%CI: 0.72-0.96, S: 80%, Sp: 80%), this parameter was lower in the second set of patients (AUC: 0.64, 95% CI: 0.52-0.75, S: 72%, Sp: 57%).

In this study, the inventors performed genome-wide miRNA profiling by microarrays in plasma samples from patients with CRC or AA, and healthy individuals. These results show that plasma miRNA expression can discriminate between patients with colorectal neoplasia and control subjects, suggesting its potential value for non-invasive detection of these lesions. To our knowledge, this is the first report analyzing genome-wide expression of plasma miRNAs in patients with CRC and AA by a high-throughput technology and, then, validating the results in an external, independent cohort. Based on this high-throughput analysis, we have identified a large number of putative plasma miRNA biomarkers useful to detect patients harboring CRC or AA. Moreover, we have confirmed the high capacity of discriminating between CRC and healthy individuals of six plasma microRNAs in a different cohort and using a different technology. It is worth mentioning that five of these miRNAs (i.e. miR19a, miR19b, miR18a, miR15b, and miR335) represent novel biomarkers, not previously reported.

The recent discovery of stable miRNAs in plasma and other fluids has opened up the possibility of using these molecules as biomarkers of disease, and several studies have evaluated this strategy in different settings. In human cancer, this approach is really promising because analysis of tumor-related circulating miRNAs could probably be able to detect neoplasia in a non-invasive fashion. However, whereas most miRNA expression profiling studies in cancer have been done using tissue samples, only a limited number of them have been focused on the potential value of circulating miRNAs in diagnosis and prognosis [21-23]. Remarkably, the use of miRNAs as biomarkers seems to be a better strategy than RNA or protein markers owing to the high stability of these molecules, even in the presence of RNAses[23].

So far, only a few studies have analyzed the expression of some candidate plasma miRNAs in CRC. Initially, Ng et al. found by qRT-PCR analysis that two plasma miRNAs (i.e. miR17-3p, and miR92a) were significantly elevated in CRC patients compared to control subjects. However, their initial analysis was limited to 95 miRNAs in 10 plasma samples (five CRC patients and five healthy individuals). More important, this study only evaluated the potential utility of plasma miRNAs in the diagnosis of CRC but not in the identification of precursor lesions [24]. Shortly after, Huang et al. reported the potential utility of 2 plasma miRNAs -miR29a and miR92a- to detect patients with both CRC and AA. However, this qRT-PCR analysis was restricted to 12 miRNAs selected on the basis of previous reports. Pu et al. analyzed by qRT-PCR the expression of three miRNAs in plasma samples from CRC patients reporting a significant overexpression of miR221, but patients with AA were not included in this study[25]. Lastly, Cheng et al. reported a significant up-regulation of miR141 plasma levels in metastatic CRC patients after analyzing the expression of three miRNAs[26].

The study was to perform a complete profiling of circulating miRNAs by using high-throughput technology in patients with CRC and AA, in order to identify those plasma miRNAs with potential utility as biomarkers for the diagnosis of patients with these lesions. Among the 743 miRNAs analyzed, we found 95 circulating miRNAs significantly dysregulated in patients with CRC, and 125 in patients with AA, some of them showing a good discriminatory capacity. It is important to mention that among those significantly up-regulated miRNAs in the microarray analysis the present inventors not only confirmed those plasma miRNAs related to CRC in previous studies, such as miR29a, miR92a and miR141, but also reported new candidate miRNAs with potential implication in CRC carcinogenesis. Moreover, among these overexpressed miRNAs, we found all members of the miR17-92 cluster (miR17, miR92a, miR19a, miR19b, miR18a, and miR20a) and two members of the miR106b-25 cluster (miR-25 and miR93), a less extensively characterized cluster paralog to miR17-92 in mammals. These findings highlight miR17-92 cluster as a central player in colorectal carcinogenesis. The miR17-92 cluster, also designated as oncomiR1, is one of the best characterized oncogenic miRNA clusters[27,28]. In that sense, it has been suggested that the increase in chromosomal instability, responsible for the progression from adenoma to adenocarcinoma, not only leads to up-regulation of oncogenes but also causes overexpression of critical miRNAs, including the miR17-92 cluster [29].

The present inventors demonstrated that some of the up-regulated miRNAs in the first set of CRC patients (i.e., miR18a, miR19a, miR19b, miR15b, miR29a and miR335) were also overexpressed in an independent cohort. Remarkably, these validated miRNAs showed a high discriminative accuracy for CRC and several combinations of these miRNAs improved the discriminative capacity of either of these miRNAs alone. Furthermore, they were able to detect early CRC (I-II) as accurately as advanced CRC (stage II-III), as well as right-sided tumors as accurately as left-sided lesions. These results point out the potential utility of these plasma miRNAs as new non-invasive biomarkers for CRC diagnosis.

Regarding colorectal cancer precursor lesions, we found that only one of the six miRNAs overexpressed in CRC (miR-18a) was confirmed to be significantly overexpressed in the two independent cohorts of patients with AA. Although miR-18a showed a quite good discriminative capacity in the first set of patients, this capacity was moderate in the second cohort. Accordingly, our results obtained in AA patients are not conclusive and further studies would be necessary to establish whether there is any miRNA alone or in combination able to detect these pre-malignant lesions accurately. In fact, in the cohort of patients with AA analyzed by Huang et al. two plasma miRs (miR-29a and miR-92a) showed quite good accuracy in discriminating AA from controls [16]. These two plasma miRNAs were also significantly overexpressed in our first cohort of AA patients but not in the second set. A potential explanation for this discrepancy between both set of patients could be the less advanced features of AA in the second cohorts of patients (Table 1). Altogether, these results indicate that evaluation of the usefulness of plasma microRNAs in patients with AA constitutes an interesting research line and deserves further investigation. Currently, detection of AA remains a major challenge for non-invasive CRC screening strategies. Indeed, it is worth mentioning that fecal immunochemical testing, a currently accepted strategy for non-invasive CRC screening in the average-risk population, shows a good performance for the diagnosis of CRC but overlook almost 50% of AA, as it has recently demonstrated in one study of our group[12].

Interestingly, all validated miRNAs in this study have been previously reported to be overexpressed in tissue samples from CRC patients [30-34]. Accordingly, it can be hypothesized that CRC constitutes the source of these plasma miRNAs. Therefore, our results not only point out miR29a, miR15b, miR19a, miR-19b, miR-18a and miR-335 as powerful biomarkers for CRC diagnosis but also highlight their implication in colorectal carcinogenesis. Moreover, considering the potential oncogenic role of these miRNAs, our results open up the possibility of future design of new targeted treatments focused on the inhibition of these molecules.

In addition to the listed microRNAs and families of microRNAs, other microRNAs may be added to enhance the detection of the colorectal cancer. For example, the method may further comprise determining of the level of expression of microRNAs that are underexpressed in colorectal neoplasia are selected from:

| |
|---|
| hsa-miR-636; |
| hsa-miR-876-3p; |
| hsa-miR-1537; |
| hsa-miR-630; |
| hsa-miR-380*; |
| hsa-miR-338-5p; |
| hsa-miR-573; |
| hsa-miR-182*; |
| hsa-miR-518c*; |
| hsa-miR-187*; |
| hsa-miR-1233; |
| hsa-miR-449b; |
| hsa-miR-1204; |
| hsa-miR-518d-3p; |
| hsa-miR-1290; |
| hsa-miR-144:9.1; |
| hsa-miR-105; |
| hsa-miR-298; |
| hsa-miR-491-5p; |
| hsa-miR-576-3p; |
| hsa-miR-590-3p; |
| hsa-miR-1257; |
| hsa-miR-1225-3p; |
| hsa-miR-127-3p; |
| hsa-miR-936; |
| hsa-miR-379; |
| hsa-miR-664*; |
| hsa-miR-548j; |
| hsa-miR-130b*; and |
| hsa-miR-515-3p. |

the method may further comprise determining of the level of expression of microRNAs that are overexpressed in colorectal neoplasia are selected from:

| |
|---|
| hsa-miR-302b; |
| hsa-miR-125a-5p; |
| hsa-miR-424; |
| hsa-miR-125b; |
| hsa-miR-100; |
| hsa-miR-768-3p:11.0; |
| hsa-miR-24; |
| hsa-miR-23a; |
| hsa-miR-1274b; |
| hsa-miR-27a; |
| hsa-miR-26b; |
| hsa-miR-30d; |
| hsa-miR-520h; |
| hsa-miR -520g; |
| hsa-miR-302^{a}; |
| hsa-miR-518c; |
| hsa-miR-335; |
| hsa-miR-29a; |
| hsa-miR-152; |
| hsa-miR-191; |
| hsa-miR-17; |
| hsa-miR-19b; |
| hsa-miR-30a; |
| hsa-miR-151-5p; |
| hsa-miR-92a; |
| hsa-miR-25; |
| hsa-miR-15b; |
| hsa-miR-15a; |
| hsa-miR-30e*; |
| hsa-miR-132*; and |
| hsa-miR-921. |

Yet another biosignature or assay will include the combination of both over and underexpressed microRNAs, e.g., from those listed hereinabove or disclosed herein. As such, the present invention also includes in on aspect the combination of both over and underexpressed microRNAs from respective microRNAs, with or without the specific listed microRNAs and families of (or co-expressed) microRNAs.

In summary, patients with CRC and AA show significantly different plasma miRNA profiles compared to healthy individuals. These tumor-related circulating miRNAs constitute novel biomarkers and represent a potential strategy for non-invasive, early diagnosis. In this study, we identify six promising candidate plasma miRNA for CRC detection. Nevertheless, this approach should be further validated in larger cohorts of patients, especially those with AA, in order to assess their efficacy and potential applicability in a screening setting.

It is contemplated that any embodiment discussed in this specification can be implemented with respect to any method, kit, reagent, or composition of the invention, and vice versa. Furthermore, compositions of the invention can be used to achieve methods of the invention.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "substantial" or "substantially" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by at least ±1, 2, 3, 4, 5, 6, 7, 10, 12 or 15%.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described defined by the appended claims.

### REFERENCES

1. Esquela-Kerscher, A. and Slack, F.J. (2006) Oncomirs - microRNAs with a role in cancer. Nat Rev Cancer, 6, 259-69.
2. Calin, G.A. and Croce, C.M. (2006) MicroRNA signatures in human cancers. Nat Rev Cancer, 6, 857-66.
3. Lu, J., Getz, G., Miska, E.A., Alvarez-Saavedra, E., Lamb, J., Peck, D., Sweet-Cordero, A., Ebert, B.L., Mak, R.H., Ferrando, A.A., Downing, J.R., Jacks, T., Horvitz, H.R. and Golub, T.R. (2005) MicroRNA expression profiles classify human cancers. Nature, 435, 834-8.
4. Schetter, A.J., Leung, S.Y., Sohn, J.J., Zanetti, K.A., Bowman, E.D., Yanaihara, N., Yuen, S.T., Chan, T.L., Kwong, D.L., Au, G.K., Liu, C.G., Calin, G.A., Croce, C.M. and Harris, C.C. (2008) MicroRNA expression profiles associated with prognosis and therapeutic outcome in colon adenocarcinoma. JAMA, 299, 425-36.
5. Mitchell, P.S., Parkin, R.K., Kroh, E.M., Fritz, B.R., Wyman, S.K., Pogosova-Agadjanyan, E.L., Peterson, A., Noteboom, J., O'Briant, K.C., Allen, A., Lin, D.W., Urban, N., Drescher, C.W., Knudsen, B.S., Stirewalt, D.L., Gentleman, R., Vessella, R.L., Nelson, P.S., Martin, D.B. and Tewari, M. (2008) Circulating microRNAs as stable blood-based markers for cancer detection. Proc Natl Acad Sci USA, 105, 10513-8.
6. Gilad, S., Meiri, E., Yogev, Y., Benjamin, S., Lebanony, D., Yerushalmi, N., Benjamin, H., Kushnir, M., Cholakh, H., Melamed, N., Bentwich, Z., Hod, M., Goren, Y. and Chajut, A. (2008) Serum microRNAs are promising novel biomarkers. PLoS One, 3, e3148.
7. Chim, S.S., Shing, T.K., Hung, E.C., Leung, T.Y., Lau, T.K., Chiu, R.W. and Lo, Y.M. (2008) Detection and characterization of placental microRNAs in maternal plasma. Clin Chem, 54, 482-90.
8. Jemal, A., Siegel, R., Ward, E., Hao, Y., Xu, J. and Thun, M.J. (2009) Cancer statistics, 2009. CA Cancer J Clin, 59, 225-49.
9. Gellad, Z.F. and Provenzale, D. Colorectal cancer: national and international perspective on the burden of disease and public health impact. Gastroenterology, 138, 2177-90.
10. Hoff, G. and Dominitz, J.A. Contrasting US and European approaches to colorectal cancer screening: which is best? Gut, 59, 407-14.
11. Whitlock, E.P., Lin, J.S., Liles, E., Beil, T.L. and Fu, R. (2008) Screening for colorectal cancer: a targeted, updated systematic review for the U.S. Preventive Services Task Force. Ann Intern Med, 149, 638-58.
12. Quintero, E., Castells, A., Bujanda, L., Cubiella, J., Salas, D., Lanas, A., Andreu, M., Carballo, F., Morillas, J.D., Hernandez, C., Jover, R., Montalvo, I., Arenas, J., Laredo, E., Hernandez, V., Iglesias, F., Cid, E., Zubizarreta, R., Sala, T., Ponce, M., Andres, M., Teruel, G., Peris, A., Roncales, M.P., Polo-Tomas, M., Bessa, X., Ferrer-Armengou, O., Grau, J., Serradesanferm, A., Ono, A., Cruzado, J., Perez-Riquelme, F., Alonso-Abreu, I., de la Vega-Prieto, M., Reyes-Melian, J.M., Cacho, G., Diaz-Tasende, J., Herreros-de-Tejada, A., Poves, C., Santander, C. and Gonzalez-Navarro, A. Colonoscopy versus fecal immunochemical testing in colorectal-cancer screening. N Engl J Med, 366, 697-706.
13. Cunningham, J.M., Oberg, A.L., Borralho, P.M., Kren, B.T., French, A.J., Wang, L., Bot, B.M., Morlan, B.W., Silverstein, K.A., Staggs, R., Zeng, Y., Lamblin, A.F., Hilker, C.A., Fan, J.B., Steer, C.J. and Thibodeau, S.N. (2009) Evaluation of a new high-dimensional miRNA profiling platform. BMC Med Genomics, 2, 57.
14. Chen, J., Lozach, J., Garcia, E.W., Barnes, B., Luo, S., Mikoulitch, I., Zhou, L., Schroth, G. and Fan, J.B. (2008) Highly sensitive and specific microRNA expression profiling using BeadArray technology. Nucleic Acids Res, 36, e87.
15. Du, P., Kibbe, W.A. and Lin, S.M. (2008) lumi: a pipeline for processing Illumina microarray. Bioinformatics, 24, 1547-8.
16. Huang, Z., Huang, D., Ni, S., Peng, Z., Sheng, W. and Du, X. Plasma microRNAs are promising novel biomarkers for early detection of colorectal cancer. Int J Cancer, 127, 118-26.
17. Chang, K.H., Mestdagh, P., Vandesompele, J., Kerin, M.J. and Miller, N. MicroRNA expression profiling to identify and validate reference genes for relative quantification in colorectal cancer. BMC Cancer, 10, 173.
18. Smyth, G.K. (2004) Linear models and empirical bayes methods for assessing differential expression in microarray experiments. Stat Appl Genet Mol Biol, 3, Article3.
19. Culhane, A.C., Thioulouse, J., Perriere, G. and Higgins, D.G. (2005) MADE4: an R package for multivariate analysis of gene expression data. Bioinformatics, 21, 2789-90.
20. Sabates-Bellver, J., Van der Flier, L.G., de Palo, M., Cattaneo, E., Maake, C., Rehrauer, H., Laczko, E., Kurowski, M.A., Bujnicki, J.M., Menigatti, M., Luz, J., Ranalli, T.V., Gomes, V., Pastorelli, A., Faggiani, R., Anti, M., Jiricny, J., Clevers, H. and Marra, G. (2007) Transcriptome profile of human colorectal adenomas. Mol Cancer Res, 5, 1263-75.
21. Lawrie, C.H., Gal, S., Dunlop, H.M., Pushkaran, B., Liggins, A.P., Pulford, K., Banham, A.H., Pezzella, F., Boultwood, J., Wainscoat, J.S., Hatton, C.S. and Harris, A.L. (2008) Detection of elevated levels of tumour-associated microRNAs in serum of patients with diffuse large B-cell lymphoma. Br J Haematol, 141, 672-5.
22. Wong, T.S., Liu, X.B., Wong, B.Y., Ng, R.W., Yuen, A.P. and Wei, W.I. (2008) Mature miR-184 as Potential Oncogenic microRNA of Squamous Cell Carcinoma of Tongue. Clin Cancer Res, 14, 2588-92.
23. Chen, X., Ba, Y., Ma, L., Cai, X., Yin, Y., Wang, K., Guo, J., Zhang, Y., Chen, J., Guo, X., Li, Q., Li, X., Wang, W., Wang, J., Jiang, X., Xiang, Y., Xu, C., Zheng, P., Zhang, J., Li, R., Zhang, H., Shang, X., Gong, T., Ning, G., Zen, K. and Zhang, C.Y. (2008) Characterization of microRNAs in serum: a novel class of biomarkers for diagnosis of cancer and other diseases. Cell Res, 18, 997-1006.
24. Ng, E.K., Chong, W.W., Jin, H., Lam, E.K., Shin, V.Y., Yu, J., Poon, T.C., Ng, S.S. and Sung, J.J. (2009) Differential expression of microRNAs in plasma of patients with colorectal cancer: a potential marker for colorectal cancer screening. Gut, 58, 1375-81.
25. Pu, X.X., Huang, G.L., Guo, H.Q., Guo, C.C., Li, H., Ye, S., Ling, S., Jiang, L., Tian, Y. and Lin, T.Y. Circulating miR-221 directly amplified from plasma is a potential diagnostic and prognostic marker of colorectal cancer and is correlated with p53 expression. J Gastroenterol Hepatol, 25, 1674-80.
26. Cheng, H., Zhang, L., Cogdell, D.E., Zheng, H., Schetter, A.J., Nykter, M., Harris, C.C., Chen, K., Hamilton, S.R. and Zhang, W. Circulating plasma MiR-141 is a novel biomarker for metastatic colon cancer and predicts poor prognosis. PLoS One, 6, e17745.
27. Mendell, J.T. (2008) miRiad roles for the miR-17-92 cluster in development and disease. Cell, 133, 217-22.
28. Olive, V., Jiang, I. and He, L. mir-17-92, a cluster of miRNAs in the midst of the cancer network. Int J Biochem Cell Biol, 42, 1348-54.
29. Diosdado, B., van de Wiel, M.A., Terhaar Sive Droste, J.S., Mongera, S., Postma, C., Meijerink, W.J., Carvalho, B. and Meijer, G.A. (2009) MiR-17-92 cluster is associated with 13q gain and c-myc expression during colorectal adenoma to adenocarcinoma progression. Br J Cancer, 101, 707-14.
30. Volinia, S., Calin, G.A., Liu, C.G., Ambs, S., Cimmino, A., Petrocca, F., Visone, R., Iorio, M., Roldo, C., Ferracin, M., Prueitt, R.L., Yanaihara, N., Lanza, G., Scarpa, A., Vecchione, A., Negrini, M., Harris, C.C. and Croce, C.M. (2006) A microRNA expression signature of human solid tumors defines cancer gene targets. Proc Natl Acad Sci USA, 103, 2257-61.
31. Monzo, M., Navarro, A., Bandres, E., Artells, R., Moreno, I., Gel, B., Ibeas, R., Moreno, J., Martinez, F., Diaz, T., Martinez, A., Balague, O. and Garcia-Foncillas, J. (2008) Overlapping expression of microRNAs in human embryonic colon and colorectal cancer. Cell Res, 18, 823-33.
32. Lanza, G., Ferracin, M., Gafa, R., Veronese, A., Spizzo, R., Pichiorri, F., Liu, C.G., Calin, G.A., Croce, C.M. and Negrini, M. (2007) mRNA/microRNA gene expression profile in microsatellite unstable colorectal cancer. Mol Cancer, 6, 54.
33. Motoyama, K., Inoue, H., Takatsuno, Y., Tanaka, F., Mimori, K., Uetake, H., Sugihara, K. and Mori, M. (2009) Over- and under-expressed microRNAs in human colorectal cancer. Int J Oncol, 34, 1069-75.
34. Earle, J.S., Luthra, R., Romans, A., Abraham, R., Ensor, J., Yao, H. and Hamilton, S.R. Association of microRNA expression with microsatellite instability status in colorectal adenocarcinoma. J Mol Diagn, 12, 433-40.

## Claims

1. A method for diagnosing or detecting colorectal neoplasia in a human subject comprising the steps of:
a. obtaining a biological sample selected from the list consisting of a plasma sample, a serum sample and a blood sample from the subject suspected of suffering from colorectal neoplasia;
b. measuring an overall expression pattern or level of a microRNAs biosignature comprising at least microRNA miR15b obtained from the biological sample/s of the subject; and
c. comparing the overall expression pattern of the microRNA biosignature from the biological sample of the subject suspected of suffering from colorectal neoplasia with the overall expression pattern of the microRNA biosignature from a biological sample of a normal subject,
wherein the normal subject is a healthy subject not suffering from colorectal neoplasia, and
wherein overexpression of at least miR15b is indicative of colorectal cancer.

2. The method of claim 1, further comprising the analysis of at least one of miR29a, miR19a, miR19b or miR335 as compared to expression from the normal subject, wherein overexpression of miRNA miR15b in combination with at least one of miR29a, miR19a, miR19b or miR335 is indicative of colorectal neoplasia.

3. The method of any of claims 1 or 2, wherein the method is capable of detecting early CRC (I-II) as accurately as advanced CRC (stage II-III), right-sided tumors and left-sided lesions.

4. A method for stratifying a patient to a subgroup at risk of suffering from colorectal neoplasia by using the method of any of claims 1 to 3.

5. A method for determining resistance or responsiveness to a colorectal neoplasia therapeutic regimen by using the method of any of claims 1-3.

6. *In vitro* use of a kit for a diagnosis of colorectal neoplasia comprising biomarker detecting reagents for determining a differential expression level of at least miR15b in a plasma sample, a serum sample or a blood sample.

7. *In vitro* use of the kit according to claim 6, which further comprises tools, vessels and reagents necessary to obtain samples from a subject selected from the group consisting of a plasma sample, a serum sample or a blood sample.

## Patentansprüche

1. Verfahren für die Diagnose oder den Nachweis von kolorektaler Neoplasie in einem menschlichen Probanden, umfassend folgende Schritte:
a. Erhalten einer biologischen Probe, ausgewählt aus der Liste bestehend aus einer Plasmaprobe, einer Serumprobe und einer Blutprobe aus dem Probanden, von dem vermutet wird, dass er an kolorektaler Neoplasie leidet;
b. Messen eines Gesamtexpressionsmusters oder -niveaus einer mikroRNA-Biosignatur umfassend mindestens mikroRNA miR15b, erhalten aus der/den biologischen Probe(n) des Probanden; und
c. Vergleichen des Gesamtexpressionsmusters der mikroRNA-Biosignatur der biologischen Probe des Probanden, von dem vermutet wird, dass er an kolorektaler Neoplasie leidet, mit dem Gesamtexpressionsmuster der mikroRNA-Biosignatur einer biologischen Probe eines normalen Probanden,
wobei der normale Proband ein gesunder Proband ist, welcher nicht an kolorektaler Neoplasie leidet, und wobei die Überexpression von mindestens miR15b indikativ für kolorektalen Krebs ist.

2. Verfahren nach Anspruch 1, ferner umfassend die Analyse mindestens einer aus miR29a, miR19a, miR19b oder miR335 im Vergleich zu der Expression des normalen Probanden, wobei die Überexpression von miRNA miR15b in Kombination mit mindestens einer aus miR29a, miR19a, miR19b oder miR335 indikativ für kolorektale Neoplasie ist.

3. Verfahren nach einem der Ansprüche 1 oder 2, wobei das Verfahren in der Lage ist, CRC im Frühstadium (I-II) so exakt wie fortgeschrittenen CRC (Stadium II-III), Tumoren der rechten Seite und Verletzungen der linken Seite nachzuweisen.

4. Verfahren zum Unterteilen eines Patienten in einer Teilgruppe, welche das Risiko hat, an kolorektaler Neoplasie zu leiden, unter Verwendung des Verfahrens nach den Ansprüchen 1 bis 3.

5. Verfahren zum Bestimmen der Widerstandsfähigkeit oder Empfindlichkeit gegenüber einem Therapieregime gegen kolorektale Neoplasie, unter Verwendung des Verfahrens nach einem der Ansprüche 1-3.

6. *In vitro*-Verwendung eines Kits für eine Diagnose von kolorektaler Neoplasie, umfassend Biomarker-Detektionsreagenzien zum Bestimmen eines differentiellen Expressionsniveaus von mindestens miR15b in einer Plasmaprobe, einer Serumprobe oder einer Blutprobe.

7. *In vitro*-Verwendung des Kits nach Anspruch 6, ferner umfassend Werkzeuge, Behälter und Reagenzien, welche nötig sind, um Proben aus einem Probanden zu erhalten, ausgewählt aus der Gruppe bestehend aus einer Plasmaprobe, einer Serumprobe oder einer Blutprobe.

## Revendications

1. Procédé de diagnostic et de détection de néoplasie colorectale chez un sujet humain comprenant les étapes de
a. obtention d'un échantillon biologique choisi parmi la liste constituée d'un échantillon de plasma, un échantillon de sérum et un échantillon de sang du sujet suspecté de souffrir de néoplasie colorectale ;
b. mesure d'un patron ou niveau d'expression globale de bio-signature de micro-ARNs comprenant au moins micro-ARN miR15b obtenu à partir d'échantillon(s) biologique(s) du sujet ; et
c. comparaison du patron d'expression globale de la bio-signature de micro-ARN de l'échantillon biologique du sujet suspecté de souffrir de néoplasie colorectale avec le patron d'expression globale de la bio-signature de micro-ARN d'un échantillon biologique d'un sujet normal
dans lequel le sujet normal est un sujet en bonne santé ne souffrant pas de néoplasie colorectale, et dans lequel l'expression globale d'au moins miR15b est indicative de cancer colorectal.

2. Procédé selon la revendication 1, comprenant en outre l'analyse d'au moins un de miR29a, miR19a, miR19b ou miR335 en comparaison avec l'expression du sujet normal, dans lequel la surexpression de miARN miR15b en combinaison avec au moins un de miR29a, miR19a, miR19b ou miR335 est indicative de néoplasie colorectale.

3. Procédé selon l'une quelconque des revendications 1 ou 2, dans lequel le procédé est apte à détecter CRC précoce (I-II) aussi précisément que CRC avancé (stade II-III), des tumeurs du côté droit et des lésions du côté gauche.

4. Procédé de stratification d'un patient dans un sous-groupe risquant de souffrir d'une néoplasie colorectale en utilisant le procédé de l'une quelconque des revendications 1 à 3.

5. Procédé de détermination de la résistance ou la sensibilité au régime thérapeutique de la néoplasie colorectale en utilisant le procédé de l'une quelconque des revendications 1-3.

6. Utilisation *in vitro* d'une trousse pour un diagnostic de néoplasie colorectale comprenant des reactifs de détection de biomarqueurs pour déterminer un niveau d'expression différentielle d'au moins miR15b dans un échantillon de plasma, un échantillon de sérum ou un échantillon de sang.

7. Utilisation *in vitro* d'une trousse selon la revendication 6, qui comprend en outre des outils, des récipients et des réactifs nécessaires pour obtenir des échantillons du sujet choisi parmi le groupe constitué d'un échantillon de plasma, un échantillon de sérum ou un échantillon de sang.
